# EUROPEAN PATENT SPECIFICATION

(11) **EP 4 417 174 B1**
(45) Date of publication and mention of the grant of the patent: **02.09.2026**
(21) Application number: 22898248.4
(22) Date of filing: 07.10.2022
(51) Int. Cl.: A61F 13/496, A61F 13/51, A61F 13/15

(54) **UNDERPANTS-TYPE DISPOSABLE WEARABLE ARTICLE**
WEARABLE-EINWEGARTIKEL VOM UNTERHOSEN-TYP
ARTICLE VESTIMENTAIRE JETABLE DU TYPE CULOTTE

(30) Priority: 29.11.2021 JP 2021193557
(43) Date of publication of application: 21.08.2024
(73) Proprietor: Daio Paper Corporation, Ehime 799-0492 (JP)
(72) Inventor: OKADA, Yuki, Shikokuchuo-shi, Ehime 799-0113 (JP); MANABE, Sadanao, Tokyo 102-0071 (JP)
(74) Representative: Williams Powell
(86) International application number: PCT/JP2022/037608
(87) International publication number: WO 2023/095464

(56) References cited:
- WO-A1-2018/092559
- WO-A1-2020/235495
- JP-A- 2013 146 419
- JP-A- 2013 146 549
- JP-A- 2015 208 534
- JP-A- 2016 047 307
- JP-A- 2016 067 436
- JP-A- 2017 131 451
- JP-A- 2019 170 831
- US-B2- 9 895 276

## Description

### Technical Field

The present invention relates to an underpants-type disposable wearing article provided with side seal regions.

### Background Art

An underpants-type disposable wearing article such as an underpants-type disposable diaper, an underpants-type disposable sanitary napkin, and an underpants-type disposable diaper cover is provided with side seal regions in which both side portions of a front body part and both side portions of a back body part are bonded to each other by welding inner surfaces thereof facing each other at welded portions scattered. Due to thus formed side seal regions, a waist opening, and leg openings are already prepared in a product state of the underpants-type disposable wearing article. Therefore, in an underpants-type disposable wearing article of this type, side seal regions protruding outward necessarily exist on an external surface of the wearing article (for example, see Patent Literature 1, 2).

Such an underpants-type disposable wearing article is unclothed after excretion or the like by tearing off the back body part and the front body part from each other at the side seal regions thereof so as to be removed from a wear's body. As a result, the side seal region is required to provide not only a peeling strength enough not to be broken during the use of the wearing article but also ease of tearing off after the use thereof. Various techniques, accordingly, have been conventionally suggested. As is commonly known, a means for tearing (peeling) off the side seal region includes interfacial peeling as well as base material-breaking (fracturing) carried out along a periphery of each welded portion.

Generally, in the side seal region, the welded portions are arranged at intervals in a predetermined pattern. With increasing of an area of each welded portion and of a ratio of a total area of the welded portions per an area of the side seal region (hereinafter, also simply referred to as an area ratio), although tearing off of the side seal region becomes more difficult, the welded portions become harder, resulting in high stability of a shape of the side seal region.

In addition, in a general underpants-type disposable wearing article, for ensuring fitting to a wear's body, in a region including a waist end portion of an outer member, a stretchable structure, which enables stretching and contracting of the region in a width direction, is provided.

In a typical example of such a stretchable structure, elongated elastic members extending along a stretchable direction are arranged at intervals between a first sheet and a second sheet overlapping with each other. Then, the first sheet and the second sheet form a planar stretchable region and serve to cover and hide the elastic members. The elastic members incorporated between the first sheet and the second sheet serve to generate a force for elastic stretch and contraction. The elastic members are fixed, at least at portions thereof located at both end portions of the stretchable region, to the first sheet and to the second sheet in a state where the elastic members are stretched in the stretchable direction. Due to this fixing, the elastic members, the first sheet, and the second sheet are integrated, thereby, the first sheet and the second sheet contract by a contraction force of the elastic members so as to form pleats (including wrinkle-shaped pleats. The pleats are formed not only in a natural length state but also in a state where the elastic members are stretched. Hereinafter the pleats and the wrinkle-shaped pleats are also simply referred to as pleats). Further, when the first sheet and the second sheet are stretched against the contraction force of the elastic members, the pleats are spread. Normally, at an elastic elongation limit, the first sheet and the second sheet are in a spread state without the pleats, then the pleats are formed in response to contraction of the elastic members, and in the natural length state, the pleats are formed so as to be arranged most densely.

In such a stretchable structure, if the first sheet and the second sheet are mutually independent, it is likely that a part or whole of one sheet may float from the other sheet and unnecessary pleats or swelling are generated, causing fitting-deterioration and positional deviation of the article during the use thereof. Accordingly, it is necessary that almost the whole range of the first sheet and that of the second sheet are bonded to each other directly or indirectly. In addition, in order to generate elasticity by the elastic members, it is necessary that the elastic members are extended over a whole length of the stretchable region in the stretchable direction and are fixed, at least at portions thereof which are located at both the end portions of the stretchable region in the stretchable direction, to the first sheet and to the second sheet such that, in response to the contraction of the elastic members, the first sheet and the second sheet are also contracted in the natural length state. That is to say, bonding between the first sheet and the second sheet as well as fixing of the elastic members to the first sheet and to the second sheet are both necessary.

Further, a bonding pattern of the first sheet and the second sheet affects shapes of the pleats. Thus, a bonding pattern has been conventionally proposed as follows. In an outer member, bonded sites, which extend in a front-back direction in such a manner as to cross elastic members, and non-bonded sites, which are continuous in the front-back direction in such a manner as to cross the elastic members, are provided in turn repeatedly in the width direction. Each bonded site has sections in which the elastic members do not cross the bonded site, and in these sections, bonded portions at which the first sheet and the second sheet are welded to each other are disposed at least at both sides of the elastic members in the front-back direction so as to closely adhere thereto, while portions at which the elastic members cross the bonded site serve as fixed portions at which the elastic members are fixed to the first sheet and to the second sheet (See Patent Literature 1 to 4). In this case, in each of the bonded sites, the bonded portions and the fixed portions are continuously arranged, and the bonded sites and the non-bonded sites are formed in turn repeatedly in the width direction. Thus, in a state where a product is contracted, portions of the first sheet which are located at the non-bonded sites and portions of the second sheet which are located at the non-bonded sites swell in opposite directions to each other to form pleats extending clearly in a direction orthogonal to the stretchable direction.

Meanwhile, in a case where such a stretchable structure is formed, for the ease of manufacturing the product, it is preferable that the first sheet, the second sheet and the elastic members extend over a whole of the side seal region and outward beyond the side seal region on a center side thereof in the width direction.

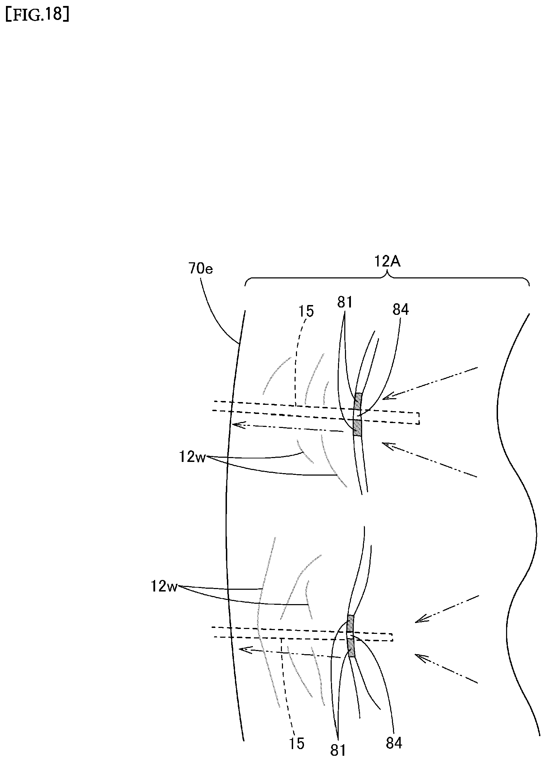

However, as shown in Fig. 18, if a side seal region 12A includes a fixed portion 84 for an elastic member 15 at a position separated laterally from an inner edge 70e of the side seal region 12A in a width direction WD, an inner portion of the side seal region 12A in the width direction WD with respect to the fixed portion 84 contracts by the elastic member 15 so as to cause unnecessary deformation in the side seal region 12A, wrinkles 12w and the like may be thereby generated. In a case where such unnecessary deformation is occurred, it is likely to happen that not only appearance but also texture and wearing feeling of the side seal region 12A are deteriorated, which is unpreferable. Note that reference sign 81 in Fig. 18 denotes a bonded portion for a first sheet and a second sheet.

### Citation List

### Patent Literature

Patent Literature 1: JP 2016-067436 A
Patent Literature 2: WO 2018-154684 A
Patent Literature 3: JP 2020-199248 A
Patent Literature 4: JP 6794546 B Patent literature 5: US 9895276 B2

### Summary of Invention

### Technical Problem

Therefore, a main object of the present invention is, for example, to reduce unnecessary deformation of a side seal region.

### Solution to Problem

Representative aspects solving the above mentioned problem are as follows.

### <First aspect>

An underpants-type disposable wearing article including:
an annular lower torso region being formed by bonding both side portions of a front body part and both side portions of a back body part, an intermediate region extending from the lower torso region of the front body part to the lower torso region of the back body part through a crotch portion, a waist opening provided on an opposite side to the intermediate region in the lower torso region, leg openings provided at both sides of the intermediate region in a width direction, an outer member forming at least the lower torso region, an inner member attached to the outer member so as to extend from a middle portion of the front body part in the width direction to a middle portion of the back body part in the width direction, and side seal regions in which both the side portions of the outer body in the front body part and both the side portions of the outer body in the back body part are bonded to each other by welding inner surfaces thereof facing each other at welded portions scattered,
wherein the outer member includes a first sheet made of a nonwoven fabric, a second sheet made of a nonwoven fabric, and plural elongated elastic members extending along the width direction to be arranged at intervals in a front-back direction between the first sheet and the second sheet;
the first sheet, the second sheet and the elastic members extend over a whole of the side seal region and outward beyond the side seal region on a center side thereof in the width direction;
in the outer member, bonded sites, which extend in the front-back direction in such a manner as to cross the elastic members, and non-bonded sites, which extend in the front-back direction in such a manner as to cross the elastic members, are provided in turn repeatedly in the width direction;
each bonded site has sections in which the elastic members do not cross the bonded site, and in these sections, bonded portions at which the first sheet and the second sheet are welded to each other are disposed at least at both sides of the elastic members in the front-back direction so as to closely adhere thereto, while portions at which the elastic members cross the bonded site serve as fixed portions at which the elastic members are fixed to the first sheet and to the second sheet;
either one or both of the side seal regions includes the fixed portion separated laterally from an inner edge of the side seal region in the width direction and a group of the welded portions, which is at least partly included in a rectangular-shaped range having a length in the front-back direction of 1.5 mm each upward and downward from the fixed portion;
the plural welded portions are arranged in the width direction such that when the group of the welded portions, which is at least partly included in the rectangular-shaped range, is viewed along the front-back direction, a non-welded portion has a dimension in the width direction of less than 1.5 mm; and
a maximum diameter of each welded portion is 0.5 to 2.0 mm and an area ratio of the welded portions to the side seal region is 5 to 30%.

### (Effect)

The side seal region of the underpants-type disposable wearing article includes the fixed portion separated laterally from the inner edge of the side seal region in the width direction but includes also the group of the welded portions, which is at least partly included in the rectangular-shaped range having a length in the front-back direction of 1.5 mm each upward and downward from the fixed portion. Further, the plural welded portions are arranged relatively densely in the width direction such that when the group of the welded portions is viewed along the front-back direction, the non-welded portion has a dimension in the width direction of less than 1.5 mm. In addition, rigidity of the welded portion is significantly higher compared to that of the non-welded portion. Therefore, even if a contraction force of an elastic member is applied to an inner portion of the side seal region with respect to the fixed portion in the width direction, deformation can be inhibited by the welded portions arranged relatively densely (so as to have a high rigidity) in and near the inner portion.

If only high rigidity of the side seal region is required, it is enough only to increase an area of each welded portion and an area ratio of the welded portions to the side seal region, but in this case, not only the side seal region becomes hard but also it becomes difficult to tear off the side seal region after the use of the wearing article. Therefore, it is important to adopt the above mentioned arrangement of the welded portions with respect to the elastic members, while the maximum diameter of each welded portion and the area ratio of the welded portions to the side seal region should fall within the above ranges.

Note that the inner edge of the side seal region in the width direction refers to an imaginary line which extends linearly in the front-back direction through an innermost position of an outer circumferential edge in the width direction of a welded portion located innermost in the width direction. It is needless to say that a side edge, an upper edge, and a lower edge of the side seal region are identical with the edges of the first sheet and those of the second sheet.

### <Second aspect>

The underpants-type disposable wearing article according to the first aspect,
wherein the plural welded portions are arranged in the front-back direction such that when a whole of the side seal region is viewed along the width direction, a dimension of the non-welded portion in the front-back direction is shorter than a dimension of the elastic member in the front-back direction in a spread state.

### (Effect)

According to the second aspect, since at least one welded portion exists in a position through which the elastic member passes in the side seal region, a target to which the contraction force of the elastic member is applied directly is decreased by a space occupied by the existing welded portion in the side seal region, thereby, wrinkles formed by the contraction force of the elastic member can be reduced in the side seal region.

Further, if fixing of the elastic member by the fixed portion of the outer member is insufficient in the side seal region, it is likely to happen that an end portion of the elastic member may fall out from the side seal region toward the center in the width direction, causing damage to elasticity of a portion outward beyond the side seal region on the center side thereof in the width direction. Contrary to this, according to the second aspect, since at least one welded portion exists in the position through which the elastic member passes in the side seal region, the welded portion crossing the elastic member can assist the fixing of the elastic member. Therefore, even if fixing of the elastic member by the fixed portion of the outer member is insufficient in the side seal region, it is unlikely to happen that the end portion of the elastic member falls out from the side seal region toward the center in the width direction.

### <Third aspect>

The underpants-type disposable wearing article according to the second aspect,
wherein, array-units of the welded portions are provided repeatedly at intervals in the front-back direction, and in each of the array-units, the welded portions being arranged at intervals along a wave shaped curve having an oscillation-center line along the width direction and a wavelength shorter than a dimension of the side seal region in the width direction,
the welded portion is provided on each point at which the wave shaped curve intersects the oscillation-center line, and at least one welded portion is provided on each side of the oscillation-center line in the front-back direction.

### (Effect)

Even if a position of the fixed portion for the elastic member of the outer member is deviated in the front-back direction or the width direction due to manufacturing reasons, in the side seal region, not only the welded portions are arranged densely near the elastic member but also the welded portions are arranged on both the sides of the elastic member in the front-back direction in a balanced way. Therefore, this third aspect is preferred.

### <Fourth aspect>

The underpants-type disposable wearing article according to the third aspect,
wherein the welded portions, which come into contact with a side edge of the side seal region, are provided repeatedly at intervals in the front-back direction.

### (Effect)

The welded portions, which come into contact with the side edge of the side seal region, are provided, thus the side seal region becomes flat to the side edge thereof. Therefore, this fourth aspect is preferred.

### <Fifth aspect>

The underpants-type disposable wearing article according to any one of the first to fourth aspects,
wherein an interval between the two adjacent bonded sites in the width direction is shorter than a dimension of the side seal region in the width direction, and
the bonded site is formed in a wave shaped curve having an oscillation-center line along the front-back direction and total amplitude smaller than the dimension of the side seal region in the width direction.

### (Effect)

If the bonded portions and the fixed portions for the elastic member are provided along the bonded site formed in such wave shaped curve, at least a part of the side seal region has necessarily the fixed portion separated laterally from the inner edge of the side seal region in the width direction. Furter, if the bonded portions and the fixed portions for the elastic member are provided along the bonded site formed in the wave shaped curve, when the inner portion of the side seal region in the width direction with respect to the fixed portion contracts by the elastic member so as to cause unnecessary deformation, the wrinkles would be generated obliquely and an area having wrinkles and an area having no wrinkles would be mixed. As a result, appearance and texture of the side seal region may be deteriorated significantly. Therefore, the above mentioned reduction of such unnecessary deformation by the welded portions is of great technical significance particularly in a case of this fifth aspect.

### Advantageous Effects of Invention

The present invention provides advantages such as reduction of unnecessary deformation of a side seal region.

### Brief Description of Drawings

Fig. 1 is a plan view illustrating an internal surface of an underpants-type disposable diaper in a spread state.
Fig. 2 is a plan view illustrating an external surface of the underpants-type disposable diaper in the spread state.
Fig. 3 is a cross-sectional view taken along 2-2 line of Fig. 1.
Fig. 4 is a cross-sectional view taken along 3-3 line of Fig. 1.
Fig. 5(a) is a cross-sectional view taken along 4-4 line of Fig. 1, and Fig. 5(b) is a cross-sectional view taken along 5-5 line of Fig. 1.
Fig. 6 is a perspective view of the underpants-type disposable diaper.
Fig. 7 is a plan view illustrating an external surface of an inner member and an outline of an outer member in the spread state.
Fig. 8 is a cross-sectional view schematically illustrating the outer member at a bonded site with exaggeratedly illustrated strong bonded portions, weak bonded portions, and non-bonded portions.
Fig. 9(a) is a plan view of an outer side portion in the example of Fig. 8, and Fig. 9(b) is a plan view of an inner side portion in the example of Fig. 8.
Fig. 10 is a cross-sectional view schematically illustrating the outer member at a bonded site with exaggeratedly illustrated strong bonded portions, weak bonded portions, and non-bonded portions.
Fig. 11(a) is a plan view of an outer side portion in the example of Fig. 10, and Fig. 11(b) is a plan view of an inner side portion in the example of Fig. 10.
Fig. 12 is a plan view illustrating an external surface of a side seal region and a region adjacent thereto in the spread state.
Fig. 13 is an enlarged view illustrating a part EV of Fig. 12.
Fig. 14 is a plan view illustrating an external surface of a side seal region and a region adjacent thereto in the spread state.
Fig. 15 is an enlarged view illustrating a part EV of Fig. 14.
Fig. 16 is a plan view illustrating an external surface of a side seal region and a region adjacent thereto in the spread state.
Fig. 17 is an enlarged view illustrating a part EV of Fig. 16.
Fig. 18 is a view for explaining a wrinkle-forming mechanism in a side seal region.

### Description of Embodiments

Hereinafter, as an example of the underpants-type disposable wearing article, an underpants-type disposable diaper will be described with reference to the accompanying drawings. Note that respective constituent members adjacent in a thickness direction are fixed or bonded to each other as necessary in a similar manner to a known diaper also at a portion other than a fixed or bonded portion described below. Each of dotted pattern regions in the cross-sectional views indicates an adhesive such as a hot melt adhesive as a fixing or bonding means. The hot melt adhesive can be applied by a known method such as slot application, bead application into a continuous line or dot line, spray application into a spiral shape, Z shape, wave shape, etc., by pattern coating (transfer of a hot melt adhesive by a letterpress method) or the like. Alternatively, a fixed portion for an elastic member is formed, instead of or in addition to the above applications of the hot melt adhesive, by application of the hot melt adhesive to an outer peripheral surface of the elastic member, and the elastic member can be fixed to a member located adjacent thereto. Examples of the hot melt adhesive include an EVA-based agent, pressure sensitive adhesive rubber-based agent (elastomer-based agent), polyolefin-based agent, and polyester/polyamide-based agent, and these can be used without particular limitation. As a fixing or bonding means for bonding respective constituent members to each other, a means by material welding such as heat sealing or ultrasonic sealing also can be used. In a portion where a pervious property in a thickness direction is required, the respective constituent members adjacent in the thickness direction are fixed or bonded to each other in an intermittent pattern. For example, when such intermittent fixing or bonding is performed with a hot melt adhesive, intermittent pattern application in a spiral shape, Z shape, wave shape, or the like can be suitably used, and when application is performed in a range larger than an application width by one nozzle, intermittent pattern application in a spiral shape, Z shape, wave shape, or the like can be performed with or without a space in a width direction. As a bonding means for bonding the respective constituent members to each other, a means by material welding such as heat sealing or ultrasonic sealing also can be used.

Note that as a nonwoven fabric in the following description, a known nonwoven fabric can be appropriately used according to a site or a purpose. Examples of a constituent fiber of the nonwoven fabric include, but are not limited to, a synthetic fiber such as a polyolefin-based fiber including polyethylene and polypropylene, polyester-based fiber, or polyamide-based fiber (including a composite fiber such as core-sheath in addition to a single component fiber), regenerated fiber such as rayon or cupra, and natural fiber such as cotton. These fibers can be mixed and used. In order to enhance flexibility of the nonwoven fabric, it is preferable to use a crimped fiber as the constituent fiber. In addition, the constituent fiber of the nonwoven fabric may be a hydrophilic fiber (including a fiber that has become hydrophilic by a hydrophilizing agent), hydrophobic fiber, or water-repellent fiber (including a fiber that has become water-repellent by a water repellent agent). In addition, the nonwoven fabric is generally classified into a short fiber nonwoven fabric, long fiber nonwoven fabric, spunbonded nonwoven fabric, meltblown nonwoven fabric, spunlace nonwoven fabric, thermal bond (air-through) nonwoven fabric, needle punch nonwoven fabric, point bond nonwoven fabric, laminated nonwoven fabric (including SMS nonwoven fabric, SMMS nonwoven fabric, or the like having a meltblown layer sandwiched between spunbonded layers), and the like depending on a fiber length, sheet forming method, fiber bonding method, and stacked structure, and any of these nonwoven fabrics can be used.

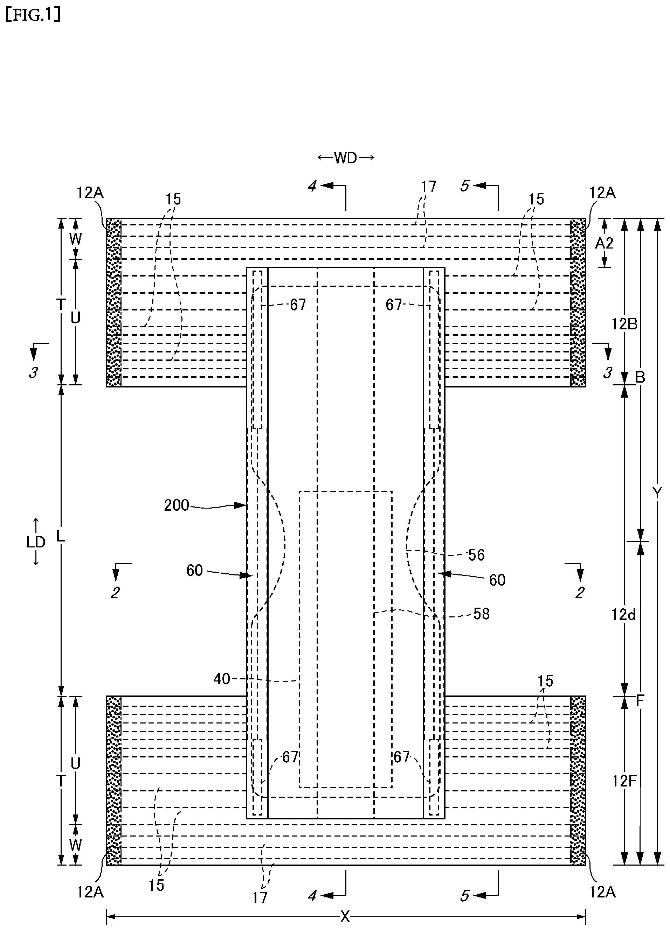

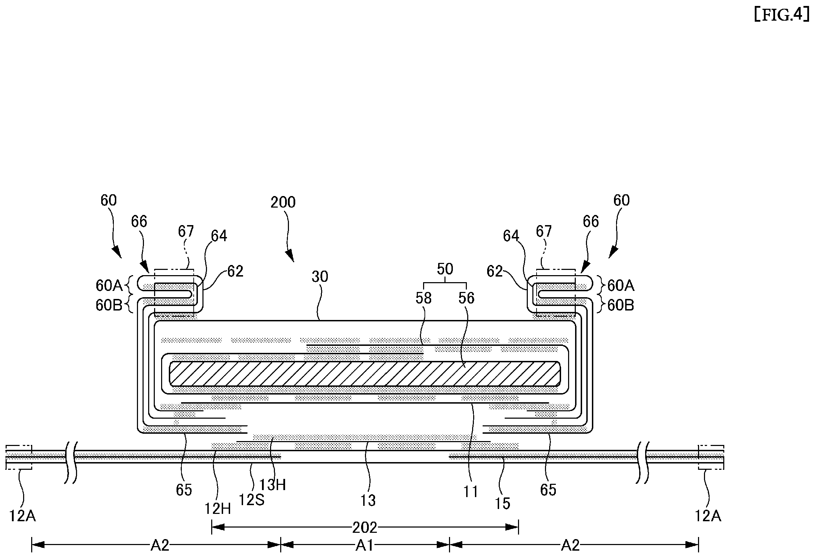

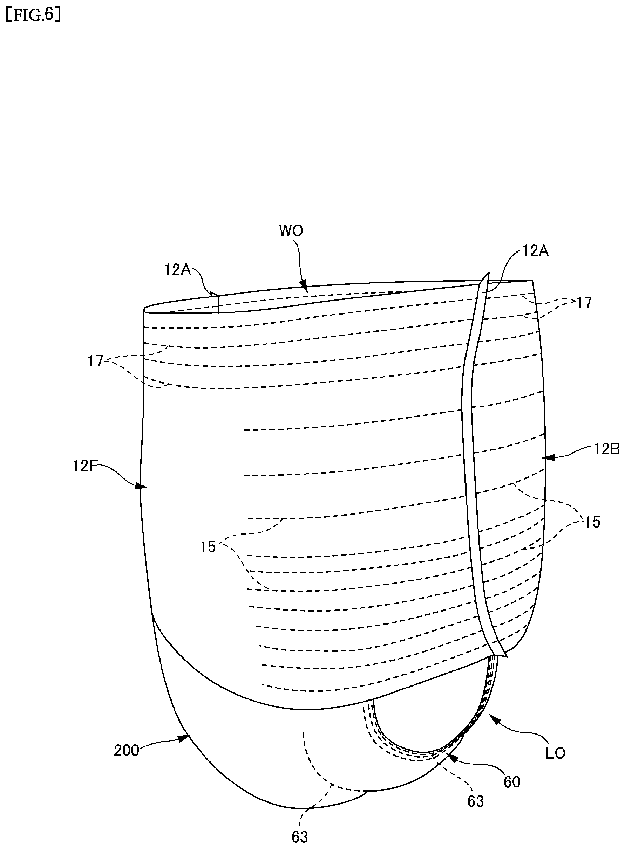

Fig. 1 to Fig. 6 illustrate an underpants-type disposable diaper. The underpants-type disposable diaper includes: a rectangular shaped front side outer member 12F forming at least a lower torso portion of a front body part F; a rectangular shaped back side outer member 12B forming at least a lower torso portion of a back body part B; and an inner member 200 provided on an inner side of an outer member 12F, 12B to extend from the front side outer member 12F to the back side outer member 12B through a crotch portion. Both side portions of the front side outer member 12F and both side portions of the back side outer member 12B are bonded to each other to form side seal regions 12A, thereby forming a waist opening WO, which is surrounded by a front end portion and a back end portion of the outer member 12A, 12B, and through which a wearer's lower torso passes, and leg openings LO, which are, on both sides of the inner member 200 in the width direction WD, surrounded by lower edges of the outer member 12F, 12B and side edges of the inner member 200, and through which wearer's legs pass, respectively. The inner member 200 is a portion for absorbing and holding excrement (e.g. urine) and the like, and the outer member 12F, 12B is a portion for supporting the inner member 200 with respect to a wearer's body. A reference character Y represents a maximum length of the diaper in a spread state (length in a front-back direction from an edge We of a waist opening WO of the front body part F to an edge We of a waist opening WO of the back body part B), and a reference character X represents the maximum width of the diaper in the spread state.

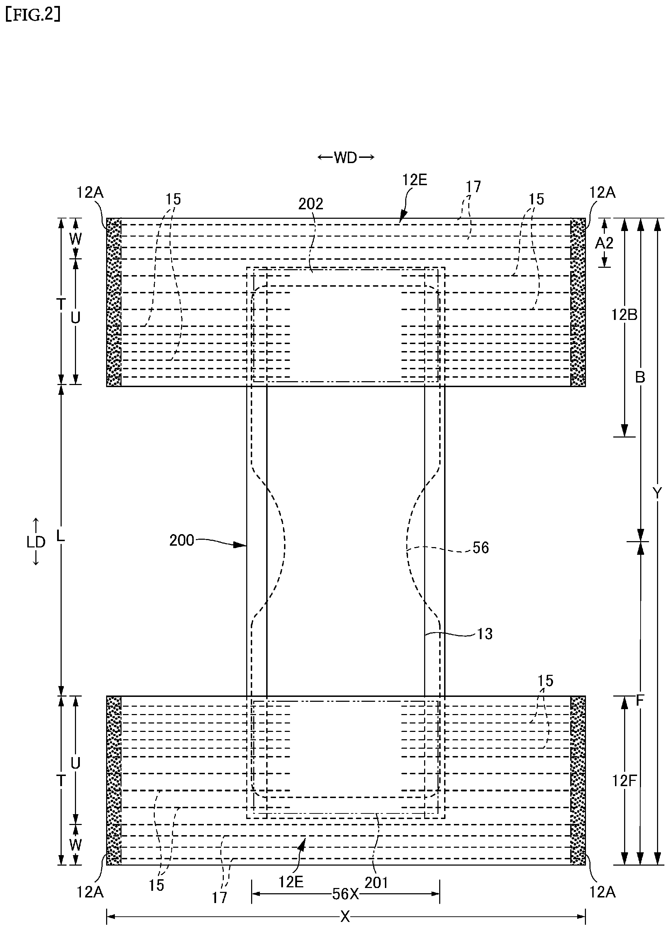

The underpants-type disposable diaper includes an annular lower torso region T being formed by bonding both the side portions of the front body part F and both the side portions of the back body part B at the side seal regions 12A, an intermediate region L extending from the lower torso region T of the front body part F to the lower torso region T of the back outer member 12F through the crotch portion, the waist opening WO provided on an opposite side to the intermediate region L in the lower torso region T, and the leg openings LO provided at both sides of the intermediate region L in the width direction WD. In other words, the lower torso region T is formed as a range in a front-back direction LD between an edge of the waist opening WO and an imaginary line extending in the width direction through upper ends of both the leg openings LO, while the intermediate region L is formed as a range in the front-back direction LD between lower ends of both the side seal regions 12A of the front body part F and lower ends of both the side seal regions 12A of the back body part **B.** In the spread state as shown in Fig. 1 and Fig. 2, both side edges of the intermediate region L are narrowed in a U shape or a curved shape to follow circumferences of the wearer's legs, and the diaper has a substantially hourglass shape as a whole.

Further, the underpants-type disposable diaper includes two main assemblies, namely the outer member 12F, 12B forming at least the lower torso region T and the inner member 200 attached to the outer member 12F, 12B so as to extend from the front body part F to the back body part B. Both the side portions of the front side outer body 12F of the front body part F and both the side portions of the back side outer body 12B of the back body part B are bonded to each other by welding inner surfaces thereof facing each other at welded portions 70 scattered in the side seal regions 12A. A dimension of the side seal region 12A in the width direction WD may be appropriately determined. For example, the dimension of the side seal region 12A in the width direction WD can be set to 10 to 20 mm.

### (Inner member)

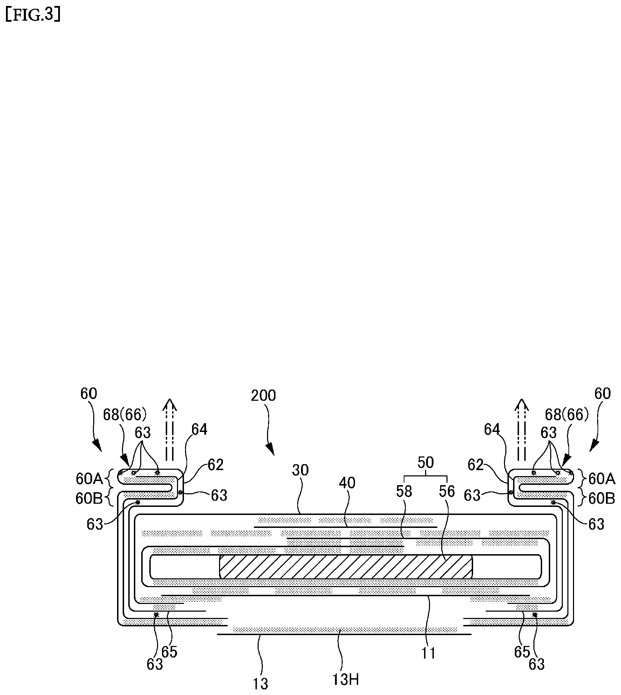

An arbitrary shape can be adopted for the inner member 200, and a rectangular shape is adopted in an illustrated example. As illustrated in Fig. 3 to Fig. 5, the inner member 200 includes a top sheet 30 corresponding to a wearer's body side, a liquid impervious sheet 11, and an absorbent element 50 interposed therebetween, and is an assembly responsible for an absorption function. Reference sign 40 denotes an intermediate sheet (second sheet) provided between the top sheet 30 and the absorbent element 50 to promptly transfer a liquid that has permeated through the top sheet 30 to the absorbent element 50, and reference sign 60 denotes rising gathers 60 extending from both side portions in the inner member 200 so as to come into contact with peripheries of the wearer's legs respectively for preventing leakage of excrement to both sides of the inner member 200.

### (Top sheet)

The top sheet 30 has a property of permeating liquid, and examples thereof may include a perforated or unperforated nonwoven fabric, a porous plastic sheet, etc. In addition, the top sheet 30 may be made of one sheet or a stacked sheet obtained by bonding two or more sheets. Similarly, the top sheet 30 may be made of one sheet or two or more sheets in a plane direction.

Both side portions of the top sheet 30 may be folded back to an underside of the absorbent element 50 at side edges thereof or may laterally protrude beyond the side edges thereof without folding back.

For the purpose of preventing positional deviation with respect to members located on an underside of the top sheet 30, and the like, it is desirable that the top sheet 30 is fixed to the members adjacent to the underside of the top sheet 30 by a bonding means based on material welding such as heat sealing or ultrasonic sealing, or with a hot melt adhesive. In the illustrated example, the top sheet 30 is fixed to a top surface of the intermediate sheet 40 and to a top surface of a portion of wrapping sheet 58 located on a top side of the absorber 56 with a hot melt adhesive applied to an underside surface of the top sheet 30.

### (Intermediate sheet)

To promptly transfer a liquid that has permeated through the top sheet 30 to the absorber 56, and to prevent a "returning" phenomenon of the absorbed liquid from the absorber 56, it is possible to provide the intermediate sheet (also referred to as the "second sheet") 40 having a higher liquid permeation rate than that of the top sheet 30. The intermediate sheet 40 can be omitted.

Examples of the intermediate sheet 40 may include a similar material to that of the top sheet 30, a spunlace nonwoven fabric, spunbonded nonwoven fabric, SMS nonwoven fabric, pulp nonwoven fabric, mixed sheet of pulp and rayon, point bond nonwoven fabric or crepe paper. In particular, an air-through nonwoven fabric is bulky, and thus is preferable. It is preferable to use a composite fiber having a core-sheath structure for the air-through nonwoven fabric. In this case, a resin used for the core may be polypropylene (PP), but is preferably polyester (PET) having high rigidity. A basis weight is preferably 17 to 80 g/m², and more preferably 17 to 50 g/m². A raw material fiber of the nonwoven fabric has preferably a fineness of 2.0 to 10 dtex. In order to make the nonwoven fabric bulky, as mixed fibers of all or a part of raw material fibers, eccentric fibers having no core in the center, hollow fibers, eccentric and hollow fibers are also preferably used.

The intermediate sheet 40 in the illustrated example is disposed at a center so as to be shorter than the width of the absorber 56, but may be disposed over the maximum width. The length of the intermediate sheet 40 in the front-back direction may be the same as the maximum length of the diaper, may be the same as the length of the absorbent element 50, or may be within a short length range centered on a liquid receiving region.

For the purpose of preventing positional deviation with respect to a member located on an underside of the intermediate sheet 40, or the like, it is desirable that the intermediate sheet 40 is fixed to the member adjacent to the underside of the intermediate sheet 40 by a bonding means based on material welding such as heat sealing or ultrasonic sealing, or with a hot melt adhesive. In the illustrated example, the intermediate sheet 40 is fixed to the top surface of the portion of the wrapping sheet 58 located on the top side of the absorber 56 with a hot melt adhesive applied to the underside surface of the intermediate sheet 40.

### (Liquid impervious sheet)

A material of the liquid impervious sheet 11 is not particularly limited, but examples thereof may include a plastic film made of a polyolefin-based resin such as polyethylene, polypropylene, etc., laminated nonwoven fabric having a plastic film provided on a surface of a nonwoven fabric, stacked sheet in which a nonwoven fabric, etc. is overlapped with and bonded to a plastic film, and the like. A material having liquid impermeability and moisture permeability which has been favorably used from the viewpoint of prevention of stuffiness is preferably used for the liquid impervious sheet 11. A microporous plastic film, which is obtained by kneading an inorganic filler in a polyolefin-based resin such as polyethylene or polypropylene, molding the kneaded mixture into a sheet, and stretching the sheet in a monoaxial or biaxial direction, is widely used as the plastic film having moisture permeability. In addition, a nonwoven fabric using a micro-denier fiber, nonwoven fabric that has reinforced leak-proofness by reducing a space between fibers by applying heat and pressure, and a sheet, which has become liquid impervious without using a waterproof film by a method for applying a super absorbent resin, hydrophobic resin, or a water repellent agent, can be used as the liquid impervious sheet 11, and it is desirable to use a resin film to obtain sufficient bonding strength at the time of bonding to a cover nonwoven fabric 13 described below by a hot melt adhesive.

As illustrated in the drawing, the liquid impervious sheet 11 has a width that fits on the underside of the absorbent element 50. In addition, to enhance a leakage prevention property, the liquid impervious sheet 11 may wrap around both sides of the absorbent element 50 so as to be extended to both side portions of a surface of the absorbent element 50 on a side of the top sheet 30. It is appropriate that a width of this extended portion is about 5 to 20 mm on each of left and right sides.

### (Rising gathers)

The rising gathers 60 extend along both the side portions in the inner member 200 over the whole length thereof in the front-back direction LD and are provided to prevent side leakage by coming into contact with the peripheries of the wearer's legs. As necessary, the rising gathers 60 can be omitted.

Each of the rising gathers (so-called three-dimensional gathers) 60 illustrated in Fig. 1, Fig. 3, and Fig. 4 stands from each side portion in the inner member 200 toward the top side. In the rising gather 60, a root side part 60B obliquely stands toward a center in the width direction, and a tip side part 60A with respect to an intermediate portion obliquely stands outward in the width direction, but a rising mode is not limited thereto and may be appropriately changed to, for example, a rising mode in which the rising gather stands as a whole toward the center in the width direction, or the like.

More specifically, the rising gather 60 of the illustrated example are obtained by folding back a belt-shaped gather nonwoven fabric 62, which has an equal length to a length of the inner member 200 in the front-back direction, in the width direction WD in two parts at a portion corresponding to a tip, and fixing plural elongated gather elastic members 63 at intervals in the width direction WD in a stretched state along the longitudinal direction between sheets of a folded portion and vicinity thereof. In the rising gather 60, a base portion located on an opposite side from a tip portion (that is to say, the base portion corresponds to an end portion on an opposite side from the sheet-folded portion in the width direction WD) is set as a root portion 65 fixed to a side portion on an underside of the liquid impervious sheet 11 in the inner member 200, and a portion other than the root portion 65 is set as a main unit portion 66 (a portion on a side of the folded portion) extending from the root portion 65. In addition, the main unit portion 66 includes the root side part 60B extending toward the center in the width direction and the tip side part 60A folded back at a tip of the root side part 60B to extend outward in the width direction. This example corresponds to a rising gather 60 of a surface contact type, and it is also possible to adopt a rising gather 60 of a line contact type which is not folded back outward in the width direction. Further, in the main unit portion 66, both end portions in the front-back direction are set as fallen parts 67 fixed to a top surface of the side portion in the top sheet 30 in a fallen state, while an intermediate portion located therebetween in the front-back direction is set as an unfixed free part 68. The gather elastic members 63 extending along the front-back direction LD are fixed, in the stretched state, to at least a tip portion of the free part 68.

In the rising gather 60 configured as described above, a contraction force of the gather elastic members 63 acts so that both end portions thereof in the front-back direction approach to each other. Then, since both the end portions of the main unit portion 66 in the front-back direction are fixed so as not to stand, but the intermediate portion located therebetween is set as the unfixed free part 68, only this free part 68 stands to come into contact with the body side as indicated by each arrow in Fig. 3. In particular, when the root portion 65 is located on an underside of the inner member 200, the free part 68 stands so as to open outward in the width direction in the crotch portion and the vicinity thereof. Thus, the rising gather 60 comes into surface contact with the periphery of each wearer's leg, and fitting is improved.

In a bending mode in which the main unit portion 66 includes the root side part 60B extending toward the center in the width direction and the tip side part 60A folded back at the tip of the root side part 60B to extend outward in the width direction as in the rising gather 60 in the illustrated example, the tip side part 60A and the root side part 60B are bonded to each other in the fallen state in each fallen part 67, and the root side part 60B is bonded to the top sheet 30 in the fallen state. For bonding facing surfaces in the fallen part 67, it is possible to use at least one of a hot melt adhesive based on various coating methods and means based on material welding such as heat sealing, ultrasonic sealing, etc. In this case, bonding between the root side part 60B and the top sheet 30 and bonding between the tip side part 60A and the root side part 60B may be performed by the same means or different means. For example, in a preferable example, bonding between the root side part 60B and the top sheet 30 is performed by the hot melt adhesive, and bonding between the tip side part 60A and the root side part 60B is performed by the material welding.

As the gather nonwoven fabric 62, a nonwoven fabric, which is flexible and excellent in uniformity/concealing property such as a spunbonded nonwoven fabric (SS, SSS, etc.), SMS nonwoven fabric (SMS, SSMMS, etc.), meltblown nonwoven fabric, etc., can be used preferably, and as necessary, such nonwoven fabric can be made water-repellent using silicone or the like. The gather nonwoven fabric 62 has preferably a fiber basis weight of about 10 to 30 g/m². As the gather elastic member 63, a rubber thread or the like may be used. In the case of using spandex rubber thread, a fineness thereof is preferably 470 to 1,240 dtex, more preferably 620 to 940 dtex. The spandex rubber thread is fixed at a stretch rate of preferably 150 to 350%, more preferably 200 to 300%. In addition, as illustrated in the drawing, a waterproof film 64 may be interposed in the twofold gather nonwoven fabric 62. In this case, the gather nonwoven fabric 62 may be partially omitted in a portion in which the waterproof film 64 is present. Nevertheless, for making an external surface of a product have a cloth-like appearance and touch, it is necessary that an external surface at least from the base to the tip of the rising gather 60 is formed by the gather nonwoven fabric 62 as in the illustrated example.

The number of gather elastic members 63 provided in the free part of the rising gather 60 is preferably 2 to 6, more preferably 3 to 5. The interval 60d therebetween is appropriately 3 to 10 mm. In such an arrangement, the free part is likely to come into surface contact with wearer's skin in a range in which the gather elastic members 63 are disposed. Further in the free part, the gather elastic members 63 may be disposed not only on a tip side but also on a root side thereof.

In the free part 68 of the rising gather 60, for bonding an internal layer of the gather nonwoven fabric 62 and an external layer of the gather nonwoven fabric 62 to each other, and for fixing the gather elastic members 63 interposed therebetween, it is possible to use at least one of a hot melt adhesive based on various coating methods and means based on material welding such as heat sealing, ultrasonic sealing, etc. When the entire surfaces of the internal layer and the external layer of the gather nonwoven fabric 62 are bonded, flexibility is impaired. Thus, it is preferable that a portion other than a bonded portion of a gather elastic member 63 is not bonded or is weakly bonded. In the illustrated example, the hot melt adhesive is applied only to the outer peripheral surface of the gather elastic member 63 by coating means such as a comb gun or a sure-wrap nozzle and then, the hot melt adhesive-coated gather elastic member 63 is interposed between the internal layer of the gather nonwoven fabric 62 and the external layer thereof. In this way, fixing of the gather elastic member 63 to the internal layer of the gather nonwoven fabric 62 and to the external layer thereof as well as fixing of the internal layer thereof and the external layer thereof to each other are performed only with the hot melt adhesive applied to the outer peripheral surface of the gather elastic member 63.

Similarly, for fixing the waterproof film 64 and the gather nonwoven fabric 62 incorporated in the rising gather 60 and fixing the fallen part 67, it is possible to use at least one of a hot melt adhesive based on various coating methods and means based on material welding such as heat sealing, ultrasonic sealing, etc.

### (Absorbent element)

The absorbent element 50 includes the absorber 56 and the wrapping sheet 58 wrapping the entire absorber 56. The wrapping sheet 58 may be omitted.

### (Absorber)

The absorber 56 can be formed by an assembly of fibers. As this fiber assembly, it is possible to use one obtained by accumulating short fibers of fluff pulp, synthetic fibers, etc., and in addition, a filament assembly obtained by opening a tow (fiber bundle) of synthetic fibers such as cellulose acetate as necessary. A fiber basis weight may be set to, for example, about 100 to 300 g/m² in the case of accumulating fluff pulp or short fibers, and may be set to, for example, about 30 to 120 g/m² in the case of the filament assembly. In the case of a synthetic fiber, the fineness is, for example, 1 to 16 dtex, preferably 1 to 10 dtex, more preferably 1 to 5 dtex. In the case of the filament assembly, the filaments may be non-crimped fibers, and are preferably crimped fibers. A degree of crimp of the crimped fibers can be set to, for example, about 5 to 75, preferably about 10 to 50, and more preferably about 15 to 50 per 2.54 cm. In addition, crimped fibers which are uniformly crimped may be used. Further, it is preferable to disperse and retain super absorbent polymer particles in the absorber 56.

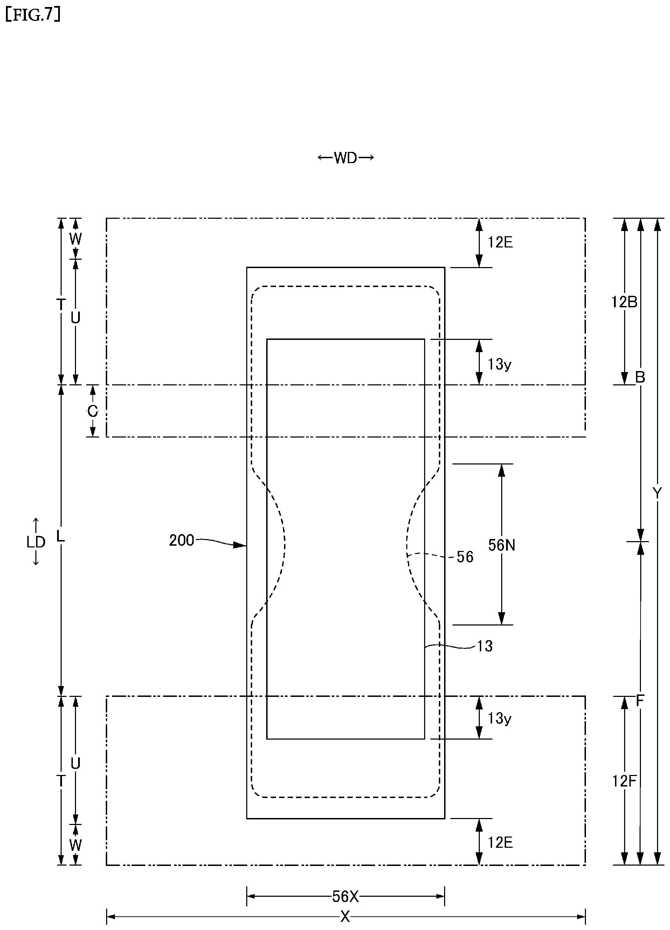

The absorber 56 may have a rectangular shape. However, as illustrated in Fig. 7 and the like, the absorber 56 has preferably an hourglass shape including, in a middle portion thereof in the front-back direction, a narrower portion 56N whose width is narrower than those of both front and back sides of the middle portion, since fitting of the absorber 56 itself and the rising gathers 60 to the peripheries of the wearer's legs is improved.

In addition, a dimension of the absorber 56 may be appropriately determined as long as the absorber 56 extends from front to back and from side to side of a ureteral outlet position, and it is preferable that the absorber 56 extends to a peripheral edge portion of the inner member 200 or a vicinity thereof in the front-back direction LD and the width direction WD. Reference sign 56X denotes a maximum width of the absorber 56.

### (Super absorbent polymer particles)

Super absorbent polymer particles may be contained in a part or a whole of the absorber 56. The super absorbent polymer particles include "powder" in addition to "particles". As the particles, those used for this type of disposable diaper may be used as they are. For example, it is desirable that the proportion of particles remaining on the sieve is 30 wt% or less by sieving (shaking for 5 minutes) using a 500 µm standard sieve (JIS Z 8801-1: 2006), and it is desirable that the proportion of particles remaining on the sieve is 60 wt% or more by sieving (shaking for 5 minutes) using a 180 µm standard sieve (JIS Z 8801-1: 2006).

As the super absorbent polymer particles, any material can be used without particular limitation, but those having a water absorption capacity of 40 g/g or more are suitable. Examples of the super absorbent polymer particles include a starch-based material, cellulose-based material, and synthetic polymer-based material. A starch-acrylic acid (salt) graft copolymer, saponified product of a starch-acrylonitrile copolymer, cross-linked product of sodium carboxymethyl cellulose, acrylic acid (salt) polymer, or the like can be used. As the shape of the super absorbent polymer particles, a usually used particulate material shape is suitable, but other shapes may also be used.

As the super absorbent polymer particles, for example, those having a water absorption speed of 70 seconds or less, particularly 40 seconds or less are suitably used. When the water absorption speed is too low, so-called returning, in which a liquid that has been supplied into the absorber 56 returns out of the absorber 56, tends to occur.

In addition, as the super absorbent polymer particles, those having a gel strength of 1000 Pa or more are preferably used. This makes it possible to effectively suppress a sticky feeling after liquid absorption, even in a case where the absorber 56 is made bulky.

A basis weight amount of the super absorbent polymer particles may be appropriately determined according to the absorption amount required for the use of the absorber 56. Therefore, although it cannot be said unconditionally, the basis weight amount may be set to 50 to 350 g/m².

### (Wrapping sheet)

When the diaper includes the wrapping sheet 58, as the material thereof, tissue paper, in particular, crepe paper, a nonwoven fabric, polyethylene-laminated nonwoven fabric, perforated sheet, or the like can be used, and sheets through which the super absorbent polymer particles will not escape are preferred. When a nonwoven fabric is used in place of crepe paper, a hydrophilic SMS (SMS, SSMMS, or the like) nonwoven fabric is particularly preferred, which may be made of polypropylene, a polyethylene/polypropylene composite material, or the like. The basis weight of the wrapping sheet 58 is preferably 5 to 40 g/m², particularly 10 to 30 g/m².

A wrapping mode with the wrapping sheet 58 may be determined appropriately, and in view of ease of manufacturing and in view of limiting escape of the super absorbent polymer particles from front and back end edges of the wrapping sheet 58, the following mode is preferable. Precisely, the wrapping sheet 58 winds around the absorber 56 in a tubular shape so as to surround a top surface, an underside surface, and both side surfaces thereof, while a front end edge portion and a back end edge portion of the wrapping sheet 58 are made to protrude forward and backward from the absorber 56, respectively. Overwrapped portions of the wrapping sheet 58 wound around the absorber 56 and overwrapped portions of the wrapping sheet 58 protruded from the absorber 56, respectively, are bonded to each other, by bonding means such as a hot melt adhesive, material welding, etc.

### (Cover nonwoven fabric)

In the outer member separated type underpants-type disposable diaper in the front-back direction, the inner member 200 is exposed between the front side outer member 12F and the back side outer member 12B, and thus it is preferable that the disposable diaper includes the cover nonwoven fabric 13 that covers an underside surface of the inner member 200 from between the front side outer member 12F and the inner member 200 to between the back side outer member 12B and the inner member 200 so as not to expose the liquid impervious sheet 11 on the underside surface of the inner member 200.

As for the cover nonwoven fabric 13, there are no particular restrictions on the type of fibers and a processing method for bonding (entanglement) of the fibers, and a similar material to a first sheet and a second sheet of the outer member 12F, 12B explained later can be appropriately selected. Nevertheless, as an example of the cover nonwoven fabric 13, an air-through nonwoven fabric can be appropriately used. In this case, it is preferable that a basis weight is 20 to 40 g/m² and a thickness is 0.3 to 1.0 mm. As the cover nonwoven fabric 13, an unperforated nonwoven fabric not having any hole penetrating the top side and underside thereof or a perforated nonwoven fabric in which a large number of holes penetrating the top side and underside thereof may be used.

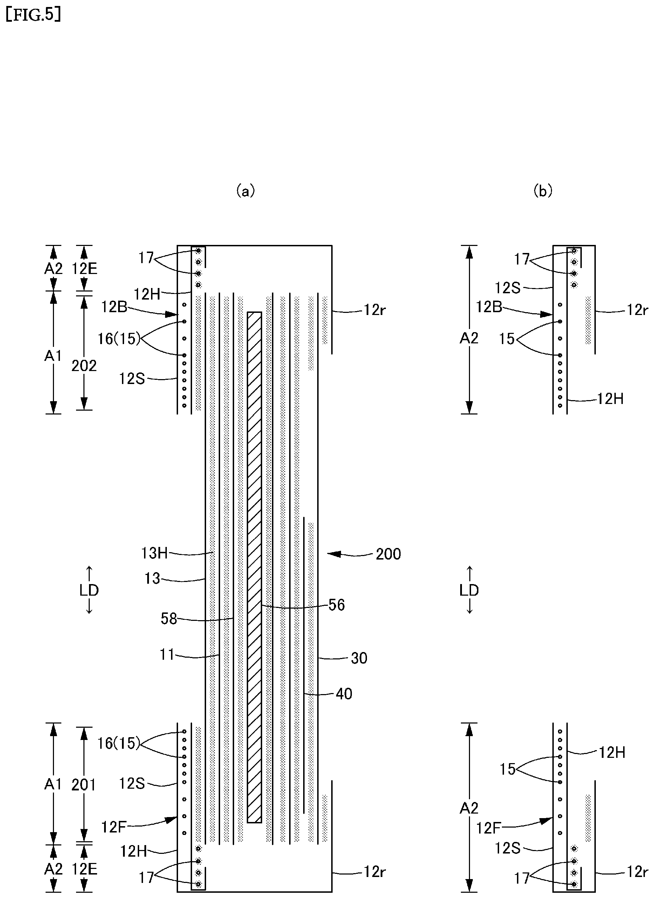

A range in the front-back direction of the cover nonwoven fabric 13 is not particularly limited. The cover nonwoven fabric 13 may extend in the front-back direction LD over a whole area from a front end to a back end of the inner member 200 as illustrated in Fig. 2 and Fig. 5. Alternatively, the cover nonwoven fabric 13 may extend in the front-back direction LD from a middle position in the front-back direction in a region overlapping the front side outer member 12F and the inner member 200 to a middle position in the front-back direction in a region overlapping the back side outer member 12B and the inner member 200 as illustrated in Fig. 7. In the example illustrated in Fig. 7, a length 13y in the front-back direction of an overlapping portion of the cover nonwoven fabric 13 and the front side outer member 12F and a length 13y in the front-back direction of an overlapping portion of the cover nonwoven fabric 13 and the back side outer member 12B may be determined as appropriate, and in a normal case, each of the lengths may be set to about 20 to 40 mm.

A range in the width direction of the cover nonwoven fabric 13 is set to a range in which an exposed portion of an underside surface of the liquid impervious sheet 11 can be concealed. For this reason, in the illustrated example, the liquid impervious sheet 11 is exposed between bases of the left and right rising gathers 60, and thus the cover nonwoven fabric 13 is provided to cover at least a range in the width direction from an underside of the base portion of one of the rising gathers 60 to an underside of the base portion of the other one of the rising gathers 60. In this way, the liquid impervious sheet 11 can be concealed by the cover nonwoven fabric 13 and the gather nonwoven fabrics 62 of the rising gathers 60. Alternatively, even when both end portions of the cover nonwoven fabric 13 in the width direction do not cover the undersides of the base portions of the rising gathers 60, but the gather nonwoven fabrics 62 cover undersides of both the end portions of the cover nonwoven fabric 13 in the width direction, the liquid impervious sheet 11 can be concealed by the cover nonwoven fabric 13 and the gather nonwoven fabrics 62. In this case, both the end portions of the cover nonwoven fabric 13 are covered with the gather nonwoven fabrics 62, and thus there is an advantage that both the side portions of the cover nonwoven fabric 13 are hardly peeled off from the liquid impervious sheet 11.

Each of the internal surface and the external surface of the cover nonwoven fabric 13 is bonded to a facing surface through the hot melt adhesive. A fixed region of the cover nonwoven fabric 13 may be set to be a whole region in the front-back direction and a whole region in the width direction of the cover nonwoven fabric 13. Alternatively, the cover nonwoven fabric 13 may be partly set to be unfixed. For example, if both the end portions of the cover nonwoven fabric 13 in the width direction are unfixed, even when side portions of the absorber 56 are somewhat contracted due to an influence of the rising gathers 60, this contraction have little effect, and wrinkles and folds are rarely formed in the cover nonwoven fabric 13, which is advantageous. A width of an unfixed part of each of both the end portions of the cover nonwoven fabric 13 in the width direction in this case may be appropriately determined and for example, the width may be set to 3 to 10 mm, preferably 5 to 8 mm.

### (Inner Member Fixing Portion)

The inner member 200 may be fixed to the outer member 12F, 12B by bonding means based on material welding such as heat sealing or ultrasonic sealing or with a hot melt adhesive. In the illustrated example, the inner member 200 is fixed to an internal surface of the outer member 12F, 12B with a hot melt adhesive applied to the underside surface of the inner member 200, that is in this case, to the underside surface of the liquid impervious sheet 11 and the root portions 65 of the rising gathers 60. Each of inner member fixing portions 201, 202 fixes the inner member 200 and each of the front side outer member 12F and back side outer member 12B to each other. Each of the inner member fixing portions 201, 202 may be provided almost entirely in an overlapping region in which the inner member 200 and each of the front side outer member 12F and back side outer member 12B overlap with each other, as illustrated in Fig. 2. For example, as each of the inner member fixing portions 201, 202, both end portions of the inner member 200 in the width direction are excluded from the overlapping region.

### (Outer member)

The outer member 12F, 12B in the illustrated example includes the rectangular shaped front side outer member 12F forming the lower torso region T of the front body part F and the rectangular shaped back side outer member 12B forming the lower torso region T of the back body part B. The front side outer member 12F and the back side outer member 12B are not continuous on a crotch side, and are separated from each other in the front-back direction LD. For example, a separation distance 12d may be set to about 0.4 to 0.5 times the maximum length Y of the diaper. The outer member 12F, 12B may be an integral unit extending continuously also in the front-back direction from the front body part F to the back body part B through the crotch portion.

In the outer member 12F, 12B, a portion located at the lower torso region T can be divided into a waist end portion W, which forms an end portion closer to the waist opening WO than another end portion, and an under-waist end portion U, which is corresponding to a portion located below the waist end portion W. In a case where the portion located at the lower torso region T has a boundary in which a stretching force in the width direction WD changes (for example, the fineness of an elastic member or the stretch rate thereof changes), a portion closer to the waist opening WO than the boundary closest to the waist opening WO is set as the waist end portion W. In a case where there is no such a boundary, a waist opening side-extended portion 12E, which extends toward the waist opening WO than the absorber 56 or the inner member 200, is set as the waist end portion W. The lengths of the above portions in the front-back direction LD vary depending on the size of a product and can be appropriately determined, but for example, the length of the waist end portion W can be 15 to 40 mm, and the length of the under-waist end portion U can be 65 to 120 mm.

In addition, in the illustrated example, the front side outer member 12F and the back side outer member 12B have the same dimension in the front-back direction LD and each of the front side outer member 12F and the back side outer member 12B does not have any portion included in the intermediate region L. However, as dash-double-dot lines illustrated in Fig. 7, the back side outer member 12B has a longer dimension in the front-back direction than the front side outer member 12F, and the front side outer member 12F does not have any portion included in the intermediate region L while the back side outer member 12B may have a gluteal cover portion C extending from the lower torso region T into the intermediate region L. It is possible to adopt a configuration in which an inguinal cover portion extending from the lower torso region T into the intermediate region L is provided also for the front side outer member 12F, and it is also possible to adopt a configuration in which the inguinal cover portion is provided for the front side outer member 12F while the gluteal cover portion is not provided for the back side outer member 12B, although these two configurations are not illustrated.

To improve fitting of the outer member 12F, 12B to a wearer's lower torso, elastic members 15 and 17 are incorporated in the outer member 12F, 12B such that a stretchable region A2, which elastically stretches and contracts in the width direction WD along with stretching and contracting of the elastic members 15 and 17, is formed in the outer member 12F, 12B. In this stretchable region A2, in a natural length state, the outer member 12F, 12B contracts in response to contraction of the elastic members such that pleats are formed. Stretch in the longitudinal direction of the elastic members 15, 17 allows stretch of the outer member 12F, 12B up to the predetermined stretch rate at which it fully extends without pleats. As the elastic members 15 and 17, it is possible to use a known elastic member such as a thread-shaped, belt-shaped elastic member without particular limitation. Synthetic rubber or natural rubber also may be used as the elastic members 15 and 17.

The elastic members 15, 17 of the illustrated example will be described in more detail. In the waist end portion W of the outer member 12F, 12B, plural waist end elastic members 17 are attached at intervals in the front-back direction to be continuous over a whole of the waist end portion W in the width direction WD. In addition, out of the waist end elastic members 17, one or plural members, which are arranged in a region adjacent to the under-waist end portion U, may overlap the inner member 200, or may be provided on both sides in the width direction of a center portion overlapping the inner member 200 in the width direction excluding the center portion itself. As the waist end elastic members 17, it is preferable to provide about two to fifteen, particularly four to ten rubber threads having a fineness of about 155 to 1,880 dtex, particularly 470 to 1,240 dtex (in the case of synthetic rubber. In the case of natural rubber, a cross-sectional area is about 0.05 to 1.5 mm², particularly 0.1 to 1.0 mm²) at intervals of 2 to 12 mm, particularly 3 to 7 mm, and it is preferable that a stretch rate of the waist end portion W in the width direction WD resulting therefrom is about 150 to 400%, particularly 220 to 320%. The intervals at which the waist end elastic members 17 are arranged in the front-back direction LD may be constant or may be changed in middle of the waist end portion W in the front-back direction LD.

In addition, it is preferable that in the under-waist end portion U of the outer member 12F, 12B, plural under-waist end elastic members 15 formed of elongated elastic members are attached at intervals in the front-back direction. As the under-waist end elastic members 15, it is preferable to provide about five to thirty rubber threads having a fineness of about 155 to 1,880 dtex, particularly 470 to 1,240 dtex (in the case of synthetic rubber. In the case of natural rubber, a cross-sectional area is about 0.05 to 1.5 mm², particularly 0.1 to 1.0 mm²) at intervals of 1 to 15 mm, particularly 3 to 8 mm, and it is preferable that a stretch rate of the under-waist end portion U in the width direction WD resulting therefrom is about 200 to 350%, particularly 240 to 300%. The intervals at which the under-waist end elastic members 15 are arranged in the front-back direction LD may be constant or may be changed in middle of the under-waist end portion U in the front-back direction LD, as in the illustrated example.

In a case in which the under-waist end elastic members 15 are provided in a range in the front-back direction having the absorber 56 as in the under-waist end portion U of the illustrated example, to prevent contraction of the absorber 56 in the width direction WD in a part or a whole of the range, a middle portion in the width direction including a part or a whole of a portion overlapping the absorber 56 in the width direction WD (preferably including the entire inner member fixing portions 201 and 202) is set as a non-stretchable region A1, and both sides thereof in the width direction are set as the stretchable region A2. The waist end portion W is preferably set as the stretchable region A2 over the whole thereof in the width direction WD, but similarly to the under-waist end portion U, the non-stretchable region A1 may be provided in middle of the waist end portion W in the width direction.

In manufacturing the outer member 12F, 12B, the stretchable region A2 and the non-stretchable region A1 can be formed by fixing the elastic members 15 to the outer member 12F, 12B, and then in a region corresponding to the non-stretchable region A1, cutting each under-waist end elastic member 15 at one place in a middle position in the width direction by pressing and heating, or cutting finely each under-waist end elastic member 15 at many places, so that elasticity of the non-stretchable region A1 is killed while elasticity of the stretchable region A2 is preserved. In these cases, in the non-stretchable region A1, cut pieces 16 and the like of the elastic members, which do not substantially contribute to the elasticity, are left in the outer member 12F, 12B.

### (Stacked Structure of Outer Member)

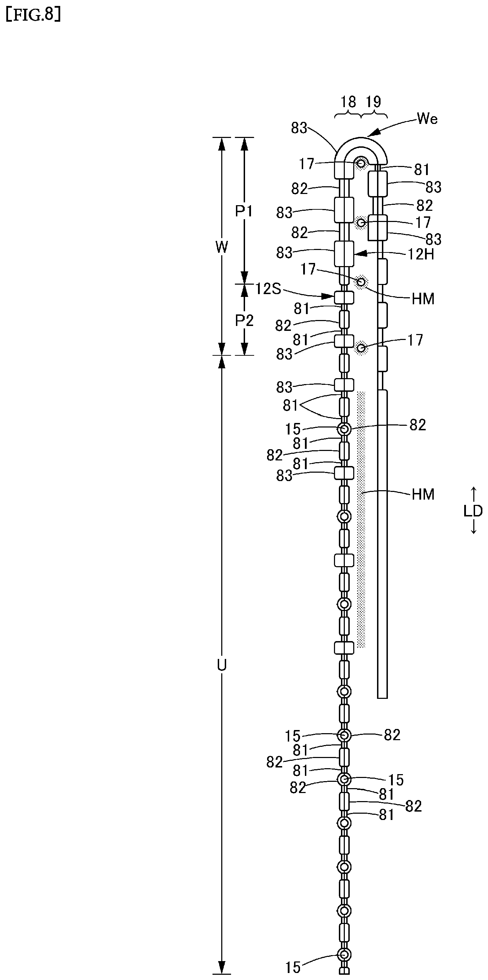

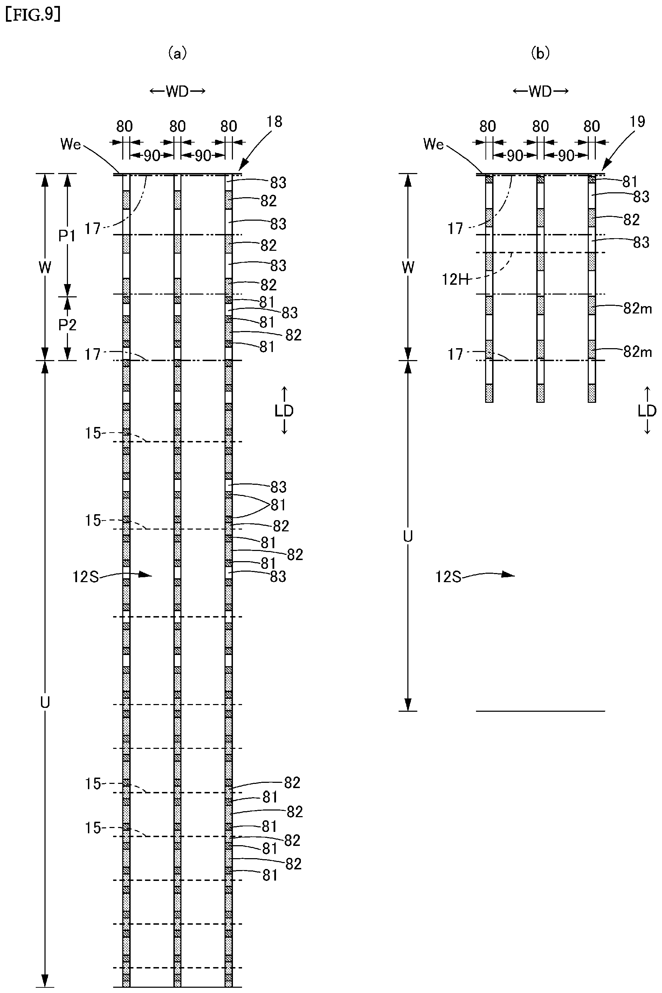

As shown in Fig. 8 and Fig. 9, the waist end portion W includes an outer side portion 18 and an inner side portion 19. The outer side portion 18 is formed by stacking the first sheet 12S made of a nonwoven fabric and the second sheet 12H made of a nonwoven fabric. The inner side portion 19 is formed by folding back inwardly the first sheet 12S and the second sheet 12H, which have been continued from the outer side portion 18, at an edge We of the waist opening WO and by further extending only the first sheet 12S out of these sheets 12S and 12H over a whole of the waist end portion W. The inner side portion 19 is bonded to the outer side portion 18 through a hot melt adhesive HM (welding can be also used for this bonding). In the illustrated example, the first sheet 12S and the second sheet 12H in the outer side portion 18 extend from the waist end portion W into the under-waist end portion U (in a case of the illustrated example, throughout the lower torso region T), but the configuration of the outer side portion is not limited thereto and may be appropriately changed. For example, the under-waist end portion U may be made of another sheet being different from the first sheet 12S and from the second sheet 12H. On the other hand, in the illustrated example, in the inner side portion 19, the second sheet 12H extends only to a middle position of the waist end portion W in the front-back direction LD while the first sheet 12S extends from the waist end portion W into the under-waist end portion U so as to cover the inner member 200 at an end portion thereof closer to the waist opening WO than another end portion, but the configuration of the inner side portion is not limited thereto. For example, both the first sheet 12S and the second sheet 12H may extend from the waist end portion W into the under-waist end portion U, both the first sheet 12S and the second sheet 12H may be within the waist end portion W, or both the first sheet 12S and the second sheet 12H may extend only to a position closer to the waist opening WO than the inner member 200 so as not to cover the end portion of the inner member 200. Further, the second sheet 12H may be provided only in the outer side portion 18 and not extend to the inner side portion 19. Moreover, unlike the illustrated example, the inner side portion 19 itself may not be provided.

A material used for the first sheet 12S and the second sheet 12H may be appropriately determined as long as it is a nonwoven fabric. For example, as the first sheet 12S and the second sheet 12H, a nonwoven fabric can be used whose constituent fiber is a synthetic fiber such as a polyolefin-based fiber including polyethylene and polypropylene, polyester-based fiber, polyamide-based fiber, mixed fiber, or composite fiber using two types or more thereof. As a processing method for bonding the fibers, for example, an air-through method, point bond method and the like can be listed. As the first sheet 12S and the second sheet 12H, a nonwoven fabric can be used whose fineness, basis weight and thickness may be appropriately determined. A preferable nonwoven fabric used for the first sheet 12S and the second sheet 12H is for example, a long fiber nonwoven fabric having a fineness of 1.0 to 2.5 dtex, a basis weight of 10 to 30 g/m² and a thickness of 0.15 to 0.50 mm.

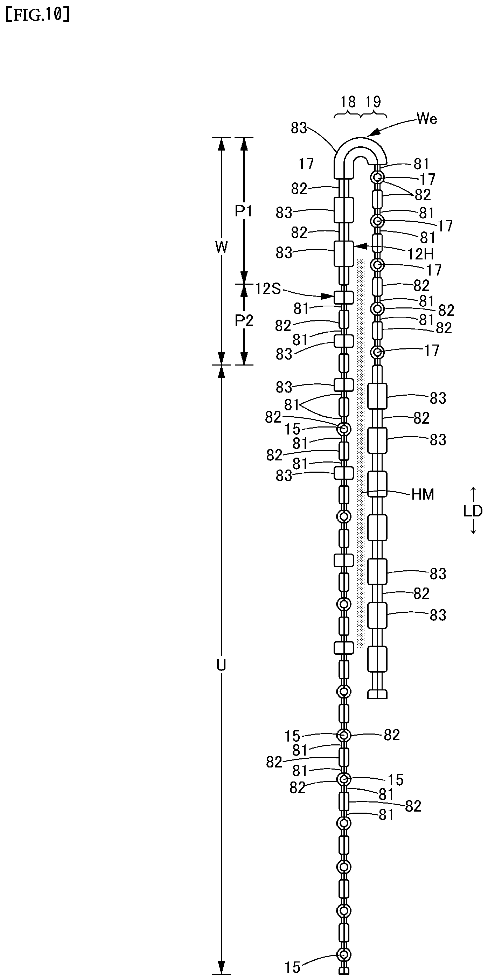

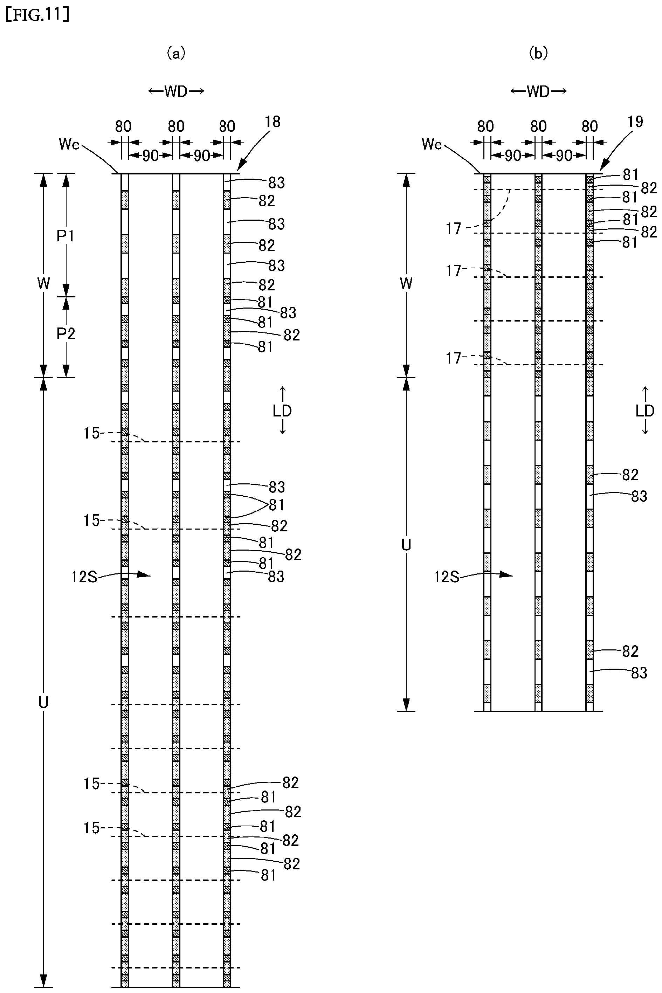

In an example illustrated in Fig. 8 and Fig. 9, the waist end elastic members 17 provided in the waist end portion W are arranged between the outer side portion 18 and the inner side portion 19, but as illustrated in Fig. 10 and Fig. 11, the waist end elastic members 17 may be arranged between the first sheet 12S and the second sheet 12H in the inner side portion 19. Further, in the example illustrated in Fig. 8 and Fig. 9, the waist end elastic members 17 provided above the middle position at which the second sheet 12H reaches are fixed between the second sheet 12H of the outer side portion 18 and the second sheet 12H of the inner side portion 19 by a hot melt adhesive HM applied to outer peripheral surfaces of the waist end elastic members 17. However, as illustrated in Fig. 10 and Fig. 11, the waist end elastic members 17 may be fixed by fixed portions 84 explained later. In the natural length state, the waist end portion W contracts in the width direction WD due to contraction of the waist end elastic members 17 such that pleats are formed, but in a wearing state where the waist end portion W stretches to some degree in the width direction WD due to stretching of the waist end elastic members 17, the pleats begin to stretch, and then in the spread state, the pleats are completely eliminated.

The under-waist end elastic members 15 provided in the under-waist end portion U are arranged between the first sheet 12S and the second sheet 12H continuing in the outer side portion 18, but an arrangement of the under-waist end elastic members 15 is not limited thereto, and it is also possible to arrange the under-waist end elastic members 15 between other sheets being different from the first sheet 12S and from the second sheet 12H. In the illustrated example, the under-waist end elastic members 15 are fixed to the first sheet 12S and to the second sheet 12H by welding at bonded sites 80 explained later, but fixing of the under-waist end elastic members 15 is not limited thereto, and the under-waist end elastic members 15 may be fixed to the first sheet 12S and to the second sheet 12H by a hot melt adhesive HM applied to the outer peripheral surfaces of the elastic members in the under-waist end portion U. In the natural length state, the under-waist end portion U contracts in the width direction WD due to contraction of the under-waist end elastic members 15 such that pleats are formed, but in the wearing state where the under-waist end portion U stretches to some degree in the width direction WD due to stretching of the under-waist end elastic members 15, the pleats begin to stretch, and then in the spread state, the pleats are completely eliminated.

In the first sheet 12S and the second sheet 12H, bonded sites 80, which extend in the front-back direction LD in such a manner as to cross the elastic members 15, 17, and non-bonded sites 90, which extend in the front-back direction LD in such a manner as to cross the elastic members 15, 17, are provided in turn repeatedly in the width direction WD. In the illustrated example, the bonded sites 80 and the non-bonded sites 90 are continuous over a substantially whole of an overlapping portion of the first sheet 12S and the second sheet 12H (in other words, over a substantially whole of the outer side portion 18 and the inner side portion 19). However, the bonded sites 80 and the non-bonded sites 90 may be provided only in a part of the overlapping portion, for example, only in the outer side portion 18, only in the under-waist end portion U in the outer side portion 18, and the like.

In each bonded site 80, in sections in which the elastic members 15, 17 do not cross the bonded site, bonded portions 81, 82 at which the first sheet 12S and the second sheet 12H are welded to each other are disposed at least at both sides of the elastic members 15, 17 in the front-back direction LD so as to closely adhere thereto, while portions at which the elastic members 15, 17 cross the bonded site serve as the fixed portions 84 at which the elastic members 15, 17 are fixed to the first sheet 12S and to the second sheet 12H. Note that a case in which the first sheet 12S and the second sheet 12H are bonded by welding includes any types of bonding modes of the first sheet 12S and the second sheet 12H as follows: a mode in which in the first sheet 12S and the second sheet 12H, substantially all fibers are molten integrally (for example, less than ten fibers may protrude) so as be film-shaped (having higher degree of transparency than surrounding); not only that, but a mode in which in both the first sheet 12S and the second sheet 12H, substantially all fibers of layers on sides of bonded surfaces thereof are welded while fibers of layers on opposite sides to the bonded surfaces thereof are not welded but left in an independent state; a mode in which in either one of the first sheet 12S and the second sheet 12H, substantially all fibers are molten integrally so as be film-shaped while in the other of the first sheet 12S and the second sheet 12H, substantially all fibers or fibers of a layer on an opposite side to the bonded surface of the other of the first sheet 12S and the second sheet 12H are not molten but left; and the like are included.

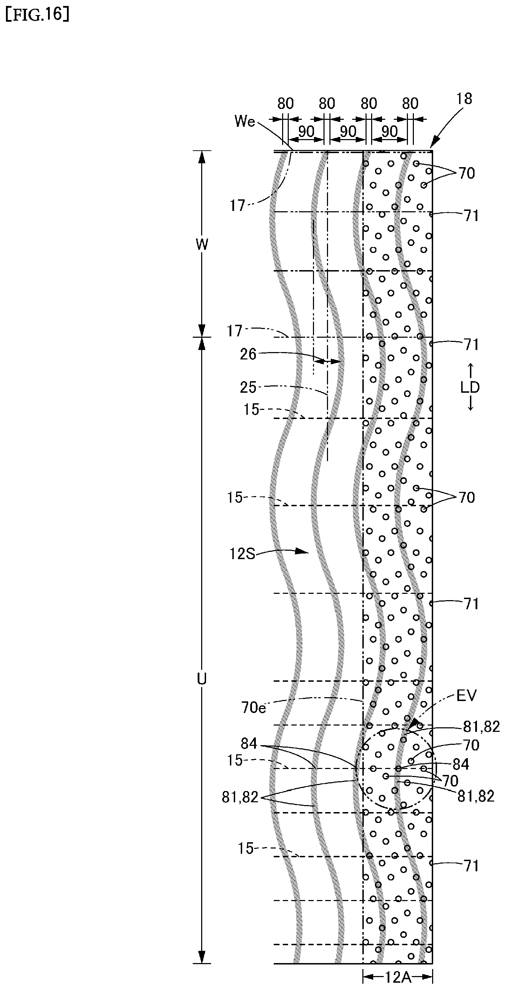

The bonded site 80 is formed in a linear shape along the front-back direction LD as in the illustrated example, in addition to this, it can be formed in a slant line being inclined at 0 to 30° in the front-back direction LD, it can be formed in a wave shape as illustrated in Fig. 16, and the like. A width of the bonded site 80 (a width of the bonded portions 81, 82) and intervals of the two adjacent bonded sites 80 in the width direction WD (a width of the non-bonded site 90) can be determined as appropriate, but for example, a width of the bonded site 80 can be about 0.1 to 5 mm, particularly about 0.5 to 3 mm and the intervals in the width direction WD can be about 3 to 10 mm, particularly about 3 to 5 mm. As a processing method for bonding by welding, ultrasonic sealing can be used and in addition to this, also heat sealing by heating a roll can be used.

As the example illustrated in Fig. 8 and Fig. 9, in a case where in the inner side portion 19, one of the first sheet 12S and the second sheet 12H extends toward a center in the front-back direction LD so as to be closer to the center than the other of the first sheet 12S and the second sheet 12H, in a thus extended portion, traces 82m similar to the bonded portions 82 may be left or may not be left at all.

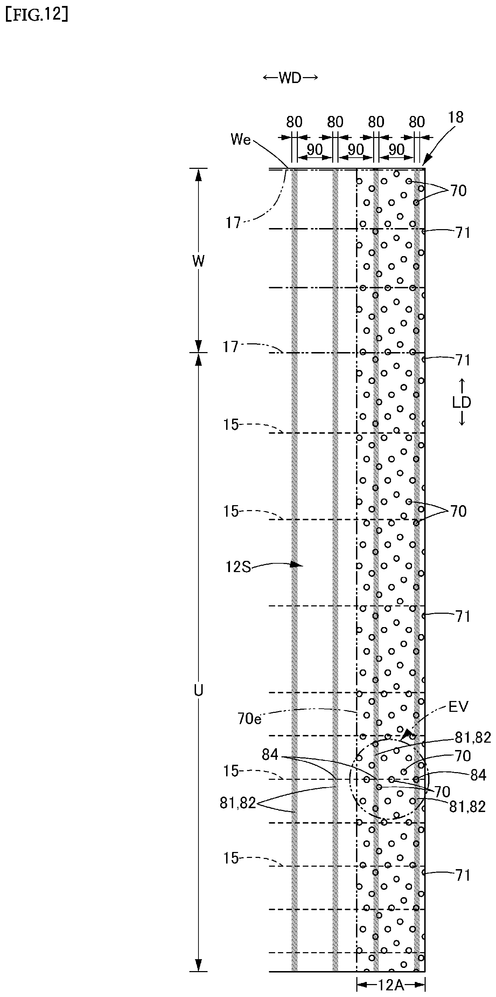

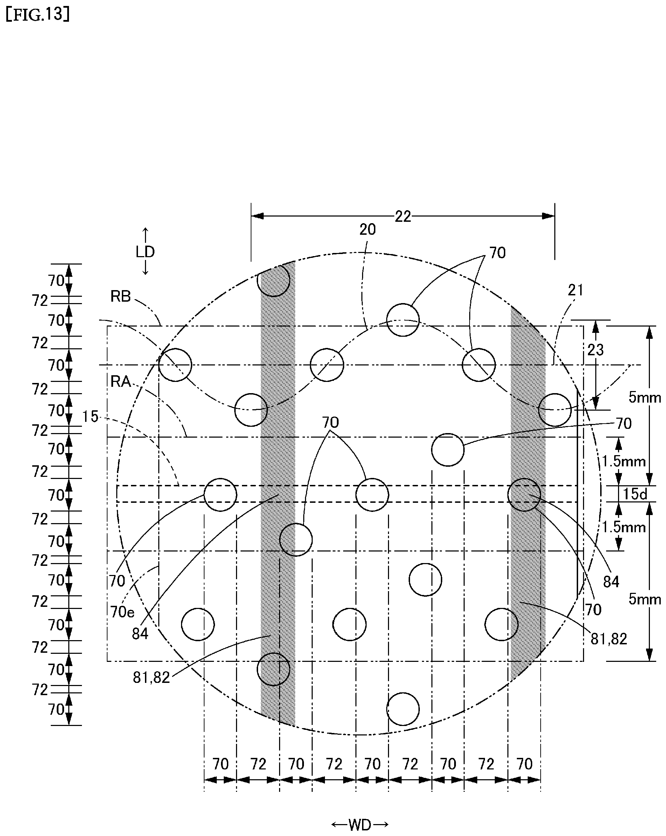

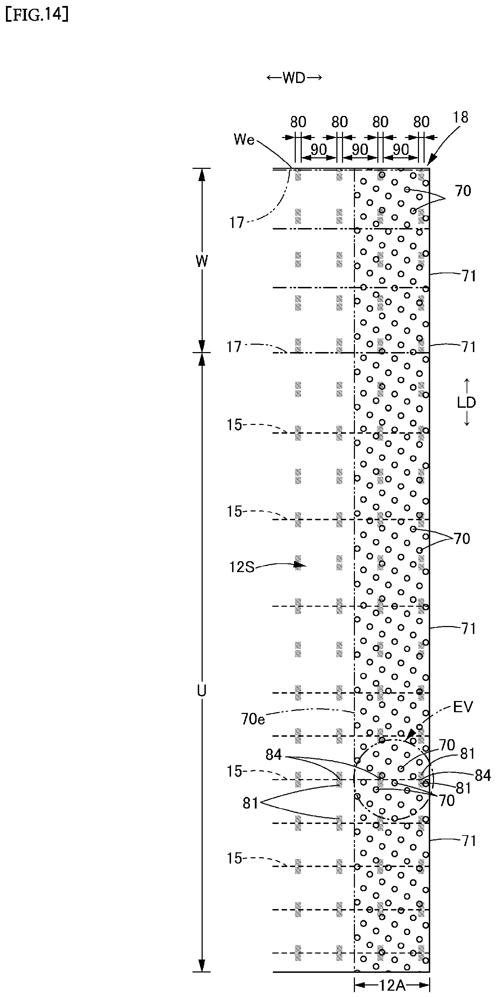

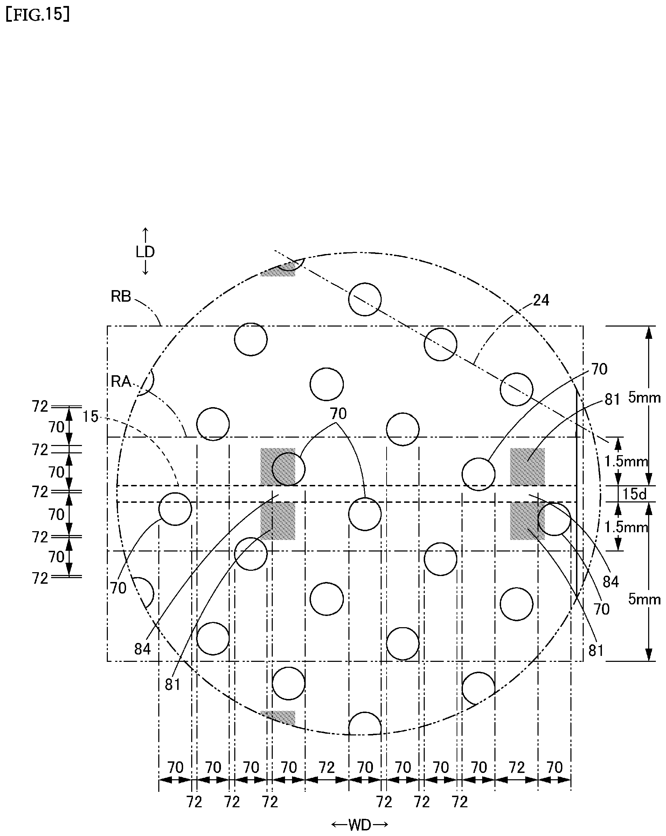

As long as the bonded portions 81, 82 are disposed at both the sides of the elastic members 15, 17 in the front-back direction so as to closely adhere thereto: as an example illustrated in Fig. 12 and Fig. 13, in all sections each of which is interposed between the two adjacent elastic members 15, 17, the bonded portions 81, 82 may be continuous throughout a whole length of the section in the front-back direction LD; as illustrated in Fig. 8 and Fig. 9, in some sections each of which is interposed between the two adjacent elastic members 15, 17, the bonded portions 81, 82 may be continuous throughout a whole length of the section in the front-back direction LD while in other sections each of which is interposed between the two adjacent elastic members 15, 17, the bonded portions 81, 82 may be discontinuous by being provided only in the vicinity of both the sides of the elastic member 15, 17 in the front-back direction; or as an example illustrated in Fig. 14 and Fig. 15, in all sections each of which is interposed between the two adjacent elastic members 15, 17, the bonded portions 81, 82 may be discontinuous by being provided only in the vicinity of both the sides of the elastic member 15, 17 in the front-back direction.

The fixed portion 84 for the elastic member 15, 17 is a cylindrical portion formed by bonding the first sheet 12S and the second sheet 12H at the bonded portion 81, 82 provided at both the sides of the elastic member 15, 17 in the front-back direction so as to closely adhere thereto. The elastic member 15, 17 is sandwiched by the cylindrical portion. The elastic member 15, 17 is fixed only by a force generated by being sandwiched by the cylindrical portion and may not be welded to the cylindrical portion. Alternatively, an outer peripheral portion of the elastic member 15, 17 may be welded to either one or both of the first sheet 12S and the second sheet 12H of the cylindrical portion.

In a case where in the section interposed between the two adjacent elastic members 15, 17, the bonded portions 81, 82 are continuous throughout a whole length of the section in the front-back direction, all of these bonded portions 81, 82 may be formed so as to have a constant bonding strength. However, it is preferable that for example, strong bonded portions 81 each having a relatively higher bonding strength are provided in the vicinity of both the sides of the elastic member 15, 17 in the front-back direction, while weak bonded portions 82 each having a relatively lower bonding strength are provided in other portions. This is because in a general nonwoven fabric, the weaker a bonding strength becomes, the harder it is to melt the fibers, and thus the more flexible the nonwoven fabric becomes. Further, if necessary, for example in a case where an interval between the two adjacent elastic members 15, 17 is wide, it is possible to provide a non-bonded portion 83 interposed between the weak bonded portions 82.

Note that being stronger or weaker (bonding strength) of the bonded portions 81, 82 means being higher or lower of tensile strength required for tearing off the bonded portions per unit area or unit length. Degree of bonding strength can be determined as appropriate, but for example, in the strong bonded portion 81, it is preferable that in the first sheet 12S and the second sheet 12H, substantially all fibers are molten integrally (for example, less than ten fibers may protrude) so as to be film-shaped (having higher degree of transparency than surrounding). On the other hand, in the weak bonded portion 82, it is preferable that a part or all of fibers located on bonded surfaces of the first sheet 12S and the second sheet 12H are welded, but a part of or all of fibers located on opposite surfaces to the bonded surfaces are not welded such that many deformable fibers are left.

Dimensions of the strong bonded portion 81, the weak bonded portion 82 and the non-bonded portion 83 can be determined as appropriate, but for example, the following ranges are preferable.
A dimension of the strong bonded portion 81 in the front-back direction LD: 2 to 4 mm
A dimension of the weak bonded portion 82 in the front-back direction LD: 1.0 to 2.0 times the dimension of the strong bonded portion 81 in the front-back direction LD
A dimension of the non-bonded portion 83 in the front-back direction LD: 0.5 to 1.2 times the dimension of the weak bonded portion 82 in the front-back direction LD

Thicknesses of the strong bonded portion 81, the weak bonded portion 82 and the non-bonded portion 83 can be determined as appropriate, but for example, the following ranges are preferable.
An apparent thickness of the weak bonded portion 82: 0.5 to 0.9 times the sum of an apparent thickness of the first sheet 12S and an apparent thickness of the second sheet 12H in the non-bonded portion 83
An apparent thickness of the strong bonded portion 81: 0.5 to 0.9 times the apparent thickness of the weak bonded portion 82

### (Arrangement of Welded Portions in Side Seal Region)

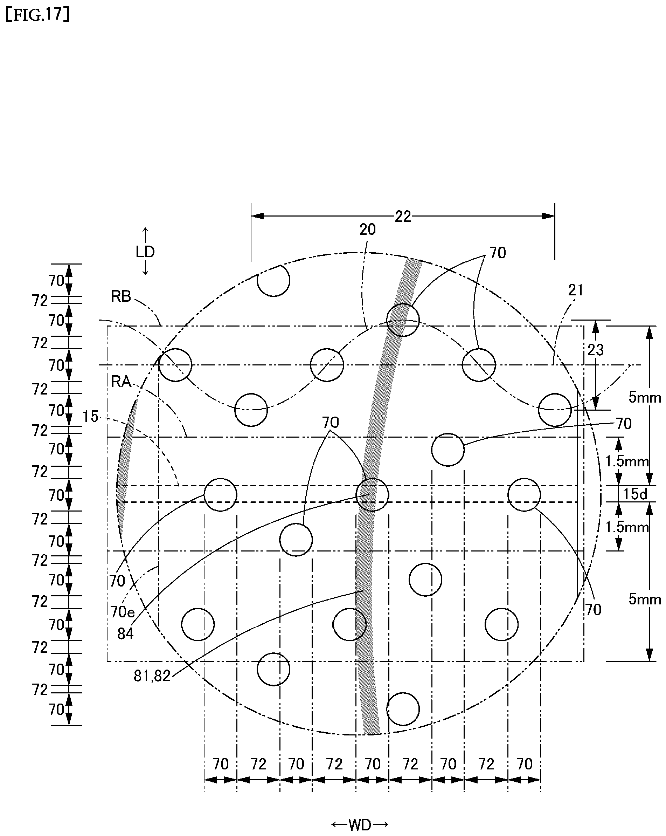

As in the example illustrated in Fig. 12 and Fig. 13, the example illustrated in Fig. 14 and Fig. 15, and the example illustrated in Fig. 16 and Fig. 17, either one or both of the side seal regions 12A preferably includes the above mentioned fixed portion 84 for the elastic member 15, 17 at a position separated laterally from an inner edge 70e of the side seal region 12A in the width direction WD. Further, it is preferable that either one or both of the side seal regions 12A includes a group of the welded portions 70, which is at least partly included in a rectangular-shaped range RA having a length in the front-back direction LD of 1.5 mm each upward and downward from the fixed portion 84, and when the group of the welded portions 70 is viewed along the front-back direction LD (see a continuity of segments designated by symbols 70, 72 on the lower part of Fig. 13), the plural welded portions 70 are arranged in the width direction WD in a manner that a non-welded portion 72 has a dimension in the width direction WD of less than 1.5 mm. In this way, even if the contraction force of the elastic member 15, 17 is applied to an inner portion of the side seal region 12A in the width direction WD with respect to the fixed portion 84, deformation can be inhibited due to the welded portions 70 arranged relatively densely (so as to have a high rigidity) in the vicinity of the inner portion. In addition, when a group of the welded portions 70, which is at least partly included in a rectangular-shaped range RB having a length in the front-back direction LD of 5 mm each upward and downward from the fixed portion 84, is viewed along the front-back direction LD, a preferable dimension of the non-welded portion 72 in the width direction WD is less than 1 mm, more preferable dimension thereof is less than 0.5 mm (as in the example illustrated in Fig. 15), particularly preferable dimension thereof is 0 mm (namely, there is no non-welded portion 72 in the range RB as in the examples illustrated in Fig. 13 and Fig. 17).

Further, when a whole of the side seal region 12A is viewed along the width direction WD (see a continuity of segments designated by symbols 70, 72 on the far-left of Fig. 13), it is preferable that the plural welded portions 70 are arranged in the front-back direction LD in a manner that a dimension of the non-welded portion 72 in the front-back direction LD is shorter than (for example, preferably less than 0.9 times, more preferably less than 0.5 times) a dimension of the elastic member 15, 17 in the spread state in the front-back direction LD (namely, a diameter of the elastic member 15, 17 when it is stretched to the spread state) (including also a case where there is no non-welded portion). In this way, since at least one welded portion 70 exists in a position through which the elastic member 15, 17 passes in the side seal region 12A, a target to which a contraction force of the elastic member 15, 17 is applied directly is decreased by a space occupied by the existing welded portion 70 in the side seal region 12A, thereby, wrinkles formed by the contraction force of the elastic member 15, 17 can be reduced in the side seal region 12A. Further, if fixing of the elastic member 15, 17 by the fixed portion 84 of the outer member 12F, 12B is insufficient in the side seal region 12A, it is likely to happen that an end portion of the elastic member 15, 17 may fall out from the side seal region 12A toward the center in the width direction WD, causing damage to elasticity of a portion outward beyond the side seal region on a center side thereof in the width direction WD. Contrary to this, if at least one welded portion 70 exists in the position through which the elastic member 15, 17 passes in the side seal region 12A, the welded portion 70 crossing the elastic member 15, 17 can assist the fixing of the elastic member 15, 17. Therefore, even if fixing of the elastic member 15, 17 by the fixed portion 84 of the outer member 12F, 12B is insufficient in the side seal region 12A, it is unlikely to happen that the end portion of the elastic member 15, 17 falls out from the side seal region 12A toward the center in the width direction WD.

Meanwhile, if only high rigidity of the side seal region 12A is required, it is enough just to increase an area of each welded portion 70 and an area ratio of the welded portions 70, but in this case, not only the side seal region 12A becomes hard but also it becomes difficult to tear off the side seal region 12A after the use of the wearing article. Therefore, it is important to adopt the above mentioned arrangement of the welded portions 70 with respect to the elastic members 15, 17, while the maximum diameter of each welded portion 70 is 0.5 to 2 mm and the area ratio of the welded portions 70 to the side seal region 12A (a total area of all welded portions 70 / an area of the side seal region 12A) is 5 to 30%. The area ratio of the welded portions 70 to the side seal region 12A is preferably 5 to 20%, in particular preferably 5 to 10%.

A planar shape of each welded portion 70 may be appropriately determined. A circle (e.g., a perfect circle and ellipse circle) as in the illustrated example is preferable, but it is possible to adopt an arbitrary shape such as a star shape, cloud shape, etc. in addition to a polygon such as a triangle, rectangle, pentagonal, etc. As the welded portion 70, a preferable ratio of a dimension in the front-back direction LD to a dimension in the width direction WD is approximately 1 (for example, less than 1.3) as that of a perfect circle, regular polygon, etc. However, as a shape of the welded portion 70, it is possible to adopt a horizontally long shape such as a rectangle having a long side along the width direction WD, and an oblique rectangle based thereon.

A dimension in the front-back direction LD and a dimension in the width direction WD of the welded portion 70 may be appropriately determined so as to be within the above mentioned range of the maximum diameter. In addition, the interval between the two adjacent welded portions 70 may be determined appropriately within the above limitation, but the minimum interval (the minimum interval among those in all directions between the circumferences of the two adjacent welded portions) is preferably within a range of 0.5 to 1 mm while the maximum interval (the maximum interval among those in all directions between the circumferences of the two adjacent welded portions) is preferably within a range of 2 to 4 mm.

An arrangement of the welded portions 70 may be determined appropriately. In a preferable example of the arrangement, as the example illustrated in Fig. 12 and Fig. 13 and as the example illustrated in Fig. 16 and Fig. 17, array units of the welded portions 70 are provided repeatedly at intervals in the front-back direction LD, and in each of the array-units, the welded portions 70 are arranged at intervals along a wave shaped curve 20 having an oscillation-center line 21 along the width direction WD and a wave length 22 being shorter than (for example, 0.5 to 0.9 times) a dimension of the side seal region 12A in the width direction WD, the welded portion 70 is provided on each point at which the wave shaped curve intersects the oscillation-center line 21, and at least one welded portion 70 is provided on each side of the oscillation-center line 21 in the front-back direction LD. By adopting such wave shaped arrangement, even if a position of the fixed portion 84 for the elastic member 15, 17 of the outer member 12F, 12B is deviated in the front-back direction LD or the width direction WD due to manufacturing reasons, in the side seal region 12A, not only the welded portions 70 are arranged densely near the elastic member 15, 17 but also the welded portions are arranged on both sides of the elastic member 15, 17 in the front-back direction in a balanced way. Therefore, this arrangement is preferred. In a case where such wave shaped arrangement is adopted, the interval between the two adjacent oscillation-center lines 21 in the front-back direction LD may be determined appropriately, but normally, the interval is preferably 0.5 times to 1 time total amplitude 23 of the wave shaped curve. In addition, the total amplitude 23 of the wave shaped curve is preferably 1 time to 5 times, particularly 1 time to 3 times the dimension of the welded portion 70 in the front-back direction LD.

As another example of the arrangement of the welded portions 70, as illustrated in Fig. 14 and Fig. 15, it is possible to adopt a diagonal striped arrangement in which rows 24 of the welded portions 70 arranged obliquely are provided repeatedly at predetermined intervals in the front-back direction LD. Additionally, it is also possible to adopt a staggered arrangement, although not illustrated.

It is preferable that in the side seal region 12A, the welded portions 71, which come into contact with the side edge of the side seal region 12A, are provided repeatedly at intervals (for example, about 3 to 12 mm) in the front-back direction LD, because in this way, the side seal region 12A becomes flat to the side edge thereof, but such welded portions, which come into contact with the side edge of the side seal region 12A, may not be provided at all.

A shape and a dimension of each welded portion 70 and an arrangement of the welded portions 70 may be constant over a whole of the side seal region 12A. Alternatively, in a first part in the side seal region 12A, a shape and a dimension of each welded portion 70 and an arrangement of the welded portions 70 are constant, whereas in a second part in the side seal region 12A, a shape and a dimension of each welded portion 70 and an arrangement of the welded portions 70 are different from those in the first part. Moreover, the welded portions 70 whose shapes, dimensions, or both of shapes and dimensions are different from one another may be mixed in a part or whole of the side seal region 12A.

On the other hand, as illustrated in Fig. 16 and Fig. 17, in a case where an interval between the two adjacent bonded sites 80 in the width direction WD is shorter than a dimension of the side seal region 12A in the width direction WD, and the bonded site 80 is formed in a wave shaped curve having an oscillation-center line 25 along the front-back direction LD and total amplitude 26 smaller than the dimension of the side seal region 12A in the width direction WD (for example, in a case where the interval between the two adjacent bonded sites 80 in the width direction WD is about 0.2 to 0.5 times the dimension of the side seal region 12A in the width direction WD, and the total amplitude 26 is about 0.1 to 0.3 times the dimension of the side seal region 12A in the width direction WD), if the bonded portions 81, 82 and the fixed portions 84 for the elastic member 15, 17 are provided along the bonded site 80 formed in the wave shaped curve, at least a part of the side seal region 12A has necessarily the fixed portion 84 at the position separated laterally from the inner edge of the side seal region 12A in the width direction WD. Furter, if the bonded portions 81, 82 and the fixed portions 84 for the elastic member 15, 17 are provided along the bonded site 80 formed in the wave shaped curve, when the inner portion of the side seal region 12A in the width direction WD with respect to the fixed portion 84 contracts by the elastic member 15, 17 so as to cause unnecessary deformation, the wrinkles would be generated obliquely and an area having wrinkles and an area having no wrinkles would be formed. As a result, appearance and texture of the side seal region may be deteriorated significantly. Therefore, the above mentioned reduction of such unnecessary deformation by the welded portions 70 is of great technical significance in a case where the bonded site 80 is formed in the wave shaped curve.

### <Description of Terms Used in Specification>

The following terms used in the specification should be understood to have the meanings defined below unless otherwise specified in the specification.

- "Front-back direction" means a direction (longitudinal direction) indicated by a reference sign LD in the drawing, "width direction" means a direction (left-right direction) indicated by a reference sign WD in the drawing, and the front-back direction and the width direction are orthogonal to each other.
- "Top side" means a side closer to wearer's skin when an underpants type disposable diaper is worn. "Underside" means a side far from wearer's skin when an underpants type disposable diaper is worn.
- "Top surface" means a surface of a member closer to wearer's skin when an underpants type disposable diaper is worn. "Underside surface" means a surface far from wearer's skin when an underpants type disposable diaper is worn.
- "Stretch rate" means a value obtained when a natural length is set to 100%. For example, the stretch rate of 200% has the same meaning as the elongation ratio of 2.
- "Gel strength" is measured as follows. 1.0 g of super absorbent polymer is added to 49.0 g of artificial urine (prepared by mixing 2wt% of urea, 0.8wt% of sodium chloride, 0.03wt% of calcium chloride dihydrate, 0.08wt% of magnesium sulfate heptahydrate, and 97.09wt% of ion exchanged water), and the resulting mixture is agitated with a stirrer. The resulting gel is left in a thermo-hygrostat at 40°C × 60%RH for three hours and then cooled to room temperature. The gel strength of the gel is measured with a curdmeter (Curdmeter-MAX ME-500 manufactured by I. Techno Engineering Co., Ltd.).
- "Basis weight" is measured as follows. After preliminary drying of a sample or a test piece, the sample or the test piece is left in a test room or a test device under normal conditions (an ambient temperature of 23 ± 1°C and with a relative humidity of 50 ± 2% at the testing site) until the weight of sample or test piece reaches constant mass. Preliminary drying is to achieve the constant mass of the sample or test piece under an environment having a temperature of 100°C. For fibers having a standard moisture regain of 0.0%, preliminary drying may be omitted. The test piece having the constant mass is cut with a cutting template having the size of 100 mm × 100 mm into samples having the size of 100 mm × 100 mm. The weight of the sample is measured. The measured weight is multiplied by 100 to determine the weight per one square meter, which is defined as the basis weight.
- The "thickness" is automatically measured under the condition of load: 0.098 N/cm² and pressure area: 2 cm² using an automatic thickness meter (KES-G5 handy compression measurement program).
- Water absorption capacity is measured in accordance with JIS K7223-1996 "Testing method for water absorption capacity of super absorbent polymers".
- Water absorption speed is defined as "time that elapses before the end point" measured with 2g of super absorbent polymers and 50g of normal saline solution in accordance with JIS K7224-1996 "Testing method for water absorption speed of super absorbent polymers".
- "Spread state" means a state where an article is spread flat without contraction (including any contraction such as contraction by an elastic member) or slackness.
- The dimension of each component means a dimension in a spread state, not in a natural length state, unless otherwise specified.

The tests and measurements are carried out in a laboratory or apparatus under normal conditions (a temperature of 23 ± 1°C and a relative humidity of 50 ± 2% at the testing site), unless the environmental condition for the tests and measurements are otherwise specified.

### Industrial Applicability

The present invention is applicable to underpants-type disposable wearing articles such as underpants-type disposable diapers and underpants-type sanitary articles.

### Reference Signs List

- 11 :: Liquid impervious sheet
- 12A :: Side seal region
- 12B :: Back side outer member
- 12E :: Waist opening side-extended portion
- 12F, 12B :: Outer member
- 12F :: Front side outer member
- 12H :: Second sheet
- 12S :: First sheet
- 13 :: Cover nonwoven fabric
- 15, 17 :: Elastic member
- 15 :: Under-waist end elastic member
- 17 :: Waist end elastic member
- 18 :: Outer side portion
- 19 :: Inner side portion
- 30 :: Top sheet
- 40 :: Intermediate sheet
- 50 :: Absorbent element
- 56 :: Absorber
- 58 :: Wrapping sheet
- 60 :: Rising gather
- 60A :: Tip side part
- 60B :: Root side part
- 62 :: Gather nonwoven fabric
- 67 :: Fallen part
- 68 :: Free part
- 70 :: Welded portion
- 72 :: Non-welded portion
- 80 :: Bonded site
- 81 :: Strong bonded portion
- 82 :: Weak bonded portion
- 83 :: Non-bonded portion
- 84 :: Fixed portion
- 90 :: Non-bonded site
- 200 :: Inner member
- 201, 202 :: Inner member fixing portion
- A1 :: Non-stretchable region
- A2 :: Stretchable region
- B :: Back body part
- C :: Gluteal cover portion
- F :: Front body part
- HM :: Hot melt adhesive
- L :: Intermediate region
- LD :: Front-back direction
- LO :: Leg opening

- RA :: Rectangular-shaped range
- T :: Lower torso region
- U :: Under-waist end portion
- W :: Waist end portion
- WD :: Width direction
- WO :: Waist opening

## Claims

1. An underpants-type disposable wearing article comprising:
an annular lower torso region (T) being formed by bonding both side portions of a front body part (F) and both side portions of a back body part (B), an intermediate region (L) extending from the lower torso region (T) of the front body part (F) to the lower torso region (T) of the back body part (B) through a crotch portion, a waist opening (WO) provided on an opposite side to the intermediate region (L) in the lower torso region (T), leg openings (LO) provided at both sides of the intermediate region (L) in a width direction (WD), an outer member (12F,12B) forming at least the lower torso region (T), an inner member (200) attached to the outer member (12F,12B) so as to extend from a middle portion of the front body part (F) in the width direction (WD) to a middle portion of the back body part (B) in the width direction (WD), and side seal regions (12A) in which both the side portions of the outer body in the front body part (F) and both the side portions of the outer body in the back body part (B) are bonded to each other by welding inner surfaces thereof facing each other at welded portions (70) scattered,
wherein the outer member (12F,12B) includes a first sheet (12S) made of a nonwoven fabric, a second sheet (12H) made of a nonwoven fabric, and plural elongated elastic members (15,17) extending along the width direction (WD) to be arranged at intervals in a front-back direction (LD) between the first sheet (12S) and the second sheet (12H);
the first sheet (12S), the second sheet (12H) and the elastic members (15,17) extend over a whole of the side seal region (12A) and outward beyond the side seal region (12A) on a center side thereof in the width direction (WD);
in the outer member (12F,12B), bonded sites (80), which extend in the front-back direction (LD) in such a manner as to cross the elastic members (15,17), and non-bonded sites (90), which extend in the front-back direction (LD) in such a manner as to cross the elastic members (15,17), are provided in turn repeatedly in the width direction (WD);
each bonded site (80) has sections in which the elastic members (15,17) do not cross the bonded site (80), and in these sections, bonded portions (81,82) at which the first sheet (12S) and the second sheet (12H) are welded to each other are disposed at least at both sides of the elastic members (15,17) in the front-back direction (LD) so as to closely adhere thereto, while portions at which the elastic members (15,17) cross the bonded site (80) serve as fixed portions (84) at which the elastic members (15,17) are fixed to the first sheet (12S) and to the second sheet (12H) ;
either one or both of the side seal regions (12A) includes the fixed portion (84) separated laterally from an inner edge of the side seal region (12A) in the width direction (WD) and a group of the welded portions (70), which is at least partly included in a rectangular-shaped range (RA) having a length in the front-back direction (LD) of 1.5 mm each upward and downward from the fixed portion (84) ;
the plural welded portions (70) are arranged in the width direction (WD) such that when the group of the welded portions (70), which is at least partly included in the rectangular-shaped range (RA), is viewed along the front-back direction (LD), a non-welded portion (72) has a dimension in the width direction (WD) of less than 1.5 mm; and
a maximum diameter of each welded portion is 0.5 to 2.0 mm and an area ratio of the welded portions (70) to the side seal region (12A) is 5 to 30%.

2. The underpants-type disposable wearing article according to claim 1,
wherein the plural welded portions (70) are arranged in the front-back direction (LD) such that when a whole of the side seal region (12A) is viewed along the width direction (WD), a dimension of the non-welded portion (72) in the front-back direction (LD) is shorter than a dimension of the elastic member (15,17) in the front-back direction (LD) in a spread state.

3. The underpants-type disposable wearing article according to claim 2,
wherein, array-units of the welded portions (70) are provided repeatedly at intervals in the front-back direction (LD), and in each of the array-units, the welded portions (70) being arranged at intervals along a wave shaped curve having an oscillation-center line along the width direction (WD) and a wavelength shorter than a dimension of the side seal region (12A) in the width direction (WD),
the welded portion is provided on each point at which the wave shaped curve intersects the oscillation-center line, and at least one welded portion is provided on each side of the oscillation-center line in the front-back direction (LD).

4. The underpants-type disposable wearing article according to claim 3
wherein the welded portions (70), which come into contact with a side edge of the side seal region (12A), are provided repeatedly at intervals in the front-back direction (LD).

5. The underpants-type disposable wearing article according to any one of claims 1 to 4,
wherein an interval between the two adjacent bonded sites (80) in the width direction (WD) is shorter than a dimension of the side seal region (12A) in the width direction (WD), and
the bonded site (80) is formed in a wave shaped curve having an oscillation-center line along the front-back direction (LD) and total amplitude smaller than the dimension of the side seal region (12A) in the width direction (WD).

## Patentansprüche

1. Höschenförmiger Wegwerf-Trageartikel, umfassend:
einen ringförmigen unteren Rumpfbereich (T), der durch Verbinden beider Seitenabschnitte eines vorderen Körperteils (F) und beider Seitenabschnitte eines hinteren Körperteils (B) gebildet ist, einen Zwischenbereich (L), der sich von dem unteren Rumpfbereich (T) des vorderen Körperteils (F) durch einen Schrittabschnitt zu dem unteren Rumpfbereich (T) des hinteren Körperteils (B) erstreckt, eine Taillenöffnung (WO), die auf einer dem Zwischenbereich (L) gegenüberliegenden Seite des unteren Rumpfbereichs (T) vorgesehen ist, Beinöffnungen (LO), die an beiden Seiten des Zwischenbereichs (L) in einer Breitenrichtung (WD) vorgesehen sind, ein Außenelement (12F, 12B), das zumindest den unteren Rumpfbereich (T) bildet, ein Innenelement (200), das an dem Außenelement (12F, 12B) so angebracht ist, dass es sich von einem mittleren Abschnitt des vorderen Körperteils (F) in der Breitenrichtung (WD) zu einem mittleren Abschnitt des hinteren Körperteils (B) in der Breitenrichtung (WD) erstreckt, sowie Seitennahtbereiche (12A), in denen beide Seitenabschnitte des Außenelements im vorderen Körperteil (F) und beide Seitenabschnitte des Außenelements im hinteren Körperteil (B) dadurch miteinander verbunden sind, dass deren einander zugewandte Innenflächen an verteilt angeordneten Schweißabschnitten (70) miteinander verschweißt werden,
wobei das Außenelement (12F, 12B) eine erste Lage (12S) aus einem Vliesstoff, eine zweite Lage (12H) aus einem Vliesstoff sowie mehrere längliche elastische Elemente (15, 17) umfasst, die sich entlang der Breitenrichtung (WD) erstrecken und in Abständen in einer Vorne-Hinten-Richtung (LD) zwischen der ersten Lage (12S) und der zweiten Lage (12H) angeordnet sind;
wobei sich die erste Lage (12S), die zweite Lage (12H) und die elastischen Elemente (15, 17) über den gesamten Seitennahtbereich (12A) sowie, an dessen zur Mitte hin gelegener Seite in der Breitenrichtung (WD), über den Seitennahtbereich (12A) hinaus erstrecken;
wobei in dem Außenelement (12F, 12B) Verbindungsstellen (80), die sich in der Vorne-Hinten-Richtung (LD) derart erstrecken, dass sie die elastischen Elemente (15, 17) kreuzen, und nicht verbundene Stellen (90), die sich in der Vorne-Hinten-Richtung (LD) derart erstrecken, dass sie die elastischen Elemente (15, 17) kreuzen, abwechselnd wiederholt in der Breitenrichtung (WD) vorgesehen sind;
wobei jede Verbindungsstelle (80) Abschnitte aufweist, in denen die elastischen Elemente (15, 17) die Verbindungsstelle (80) nicht kreuzen, und in diesen Abschnitten Verbindungsabschnitte (81, 82), an denen die erste Lage (12S) und die zweite Lage (12H) miteinander verschweißt sind, zumindest an beiden Seiten der elastischen Elemente (15, 17) in der Vorne-Hinten-Richtung (LD) so angeordnet sind, dass sie eng daran anliegen, während Abschnitte, an denen die elastischen Elemente (15, 17) die Verbindungsstelle (80) kreuzen, als Festlegungsabschnitte (84) dienen, an denen die elastischen Elemente (15, 17) an der ersten Lage (12S) und an der zweiten Lage (12H) festgelegt sind;
wobei wenigstens einer der Seitennahtbereiche (12A) den Festlegungsabschnitt (84) aufweist, der in der Breitenrichtung (WD) seitlich von einem inneren Rand des Seitennahtbereichs (12A) beabstandet ist, sowie eine Gruppe der Schweißabschnitte (70), die zumindest teilweise in einem rechteckigen Bereich (RA) enthalten ist, der eine Länge in der Vorne-Hinten-Richtung (LD) von jeweils 1,5 mm nach oben und nach unten von dem Festlegungsabschnitt (84) aufweist;
wobei die mehreren Schweißabschnitte (70) derart in der Breitenrichtung (WD) angeordnet sind, dass, wenn die Gruppe der Schweißabschnitte (70), die zumindest teilweise in dem rechteckigen Bereich (RA) enthalten ist, entlang der Vorne-Hinten-Richtung (LD) betrachtet wird, ein nicht verschweißter Abschnitt (72) eine Abmessung in der Breitenrichtung (WD) von weniger als 1,5 mm aufweist; und
wobei ein maximaler Durchmesser jedes Schweißabschnitts 0,5 bis 2,0 mm beträgt und ein Flächenanteil der Schweißabschnitte (70) an dem Seitennahtbereich (12A) 5 bis 30 % beträgt.

2. Höschenförmiger Wegwerf-Trageartikel nach Anspruch 1, wobei die mehreren Schweißabschnitte (70) derart in der Vorne-Hinten-Richtung (LD) angeordnet sind, dass, wenn der gesamte Seitennahtbereich (12A) entlang der Breitenrichtung (WD) betrachtet wird, eine Abmessung des nicht verschweißten Abschnitts (72) in der Vorne-Hinten-Richtung (LD) kürzer ist als eine Abmessung des elastischen Elements (15, 17) in der Vorne-Hinten-Richtung (LD) in einem ausgebreiteten Zustand.

3. Höschenförmiger Wegwerf-Trageartikel nach Anspruch 2, wobei Anordnungseinheiten der Schweißabschnitte (70) wiederholt in Abständen in der Vorne-Hinten-Richtung (LD) vorgesehen sind und in jeder der Anordnungseinheiten die Schweißabschnitte (70) in Abständen entlang einer wellenförmigen Kurve angeordnet sind, die eine Oszillationsmittellinie entlang der Breitenrichtung (WD) und eine Wellenlänge aufweist, die kürzer ist als eine Abmessung des Seitennahtbereichs (12A) in der Breitenrichtung (WD), wobei der Schweißabschnitt an jedem Punkt vorgesehen ist, an dem die wellenförmige Kurve die Oszillationsmittellinie schneidet, und wobei mindestens ein Schweißabschnitt auf jeder Seite der Oszillationsmittellinie in der Vorne-Hinten-Richtung (LD) vorgesehen ist.

4. Höschenförmiger Wegwerf-Trageartikel nach Anspruch 3, wobei die Schweißabschnitte (70), die mit einem Seitenrand des Seitennahtbereichs (12A) in Berührung kommen, wiederholt in Abständen in der Vorne-Hinten-Richtung (LD) vorgesehen sind.

5. Höschenförmiger Wegwerf-Trageartikel nach einem der Ansprüche 1 bis 4, wobei ein Abstand zwischen den zwei benachbarten Verbindungsstellen (80) in der Breitenrichtung (WD) kürzer ist als eine Abmessung des Seitennahtbereichs (12A) in der Breitenrichtung (WD), und wobei die Verbindungsstelle (80) in Form einer wellenförmigen Kurve ausgebildet ist, die eine Oszillationsmittellinie entlang der Vorne-Hinten-Richtung (LD) und eine Gesamtamplitude aufweist, die kleiner ist als die Abmessung des Seitennahtbereichs (12A) in der Breitenrichtung (WD).

## Revendications

1. Article jetable de type culotte destiné à être porté, comprenant :
une région annulaire du torse inférieur (T) formée par liaison des deux parties latérales d'une partie de corps avant (F) et des deux parties latérales d'une partie de corps arrière (B), une région intermédiaire (L) s'étendant depuis la région du torse inférieur (T) de la partie de corps avant (F) jusqu'à la région du torse inférieur (T) de la partie de corps arrière (B) en passant par une partie d'entrejambe, une ouverture de taille (WO) prévue du côté opposé à la région intermédiaire (L) dans la région du torse inférieur (T), des ouvertures de jambe (LO) prévues des deux côtés de la région intermédiaire (L) dans une direction de la largeur (WD), un élément extérieur (12F, 12B) formant au moins la région du torse inférieur (T), un élément intérieur (200) fixé à l'élément extérieur (12F, 12B) de manière à s'étendre depuis une partie médiane de la partie de corps avant (F) dans la direction de la largeur (WD) jusqu'à une partie médiane de la partie de corps arrière (B) dans la direction de la largeur (WD), et des régions de scellement latéral (12A) au niveau desquelles les deux parties latérales du corps extérieur dans la partie de corps avant (F) et les deux parties latérales du corps extérieur dans la partie de corps arrière (B) sont liées l'une à l'autre par soudage de leurs surfaces intérieures en regard l'une de l'autre, au niveau de parties soudées (70) réparties,
dans lequel l'élément extérieur (12F, 12B) comprend une première feuille (12S) en non-tissé, une deuxième feuille (12H) en non-tissé, et plusieurs éléments élastiques allongés (15, 17) s'étendant selon la direction de la largeur (WD) de manière à être disposés à intervalles dans une direction avant-arrière (LD) entre la première feuille (12S) et la deuxième feuille (12H) ;
la première feuille (12S), la deuxième feuille (12H) et les éléments élastiques (15, 17) s'étendent sur la totalité de la région de scellement latéral (12A) ainsi que vers l'extérieur au-delà de la région de scellement latéral (12A), du côté central de celle-ci dans la direction de la largeur (WD) ;
dans l'élément extérieur (12F, 12B), des sites liés (80), qui s'étendent dans la direction avant-arrière (LD) de manière à croiser les éléments élastiques (15, 17), et des sites non liés (90), qui s'étendent dans la direction avant-arrière (LD) de manière à croiser les éléments élastiques (15, 17), sont prévus alternativement de façon répétée dans la direction de la largeur (WD) ;
chaque site lié (80) comporte des sections dans lesquelles les éléments élastiques (15, 17) ne croisent pas le site lié (80), et dans ces sections, des parties liées (81, 82), au niveau desquelles la première feuille (12S) et la deuxième feuille (12H) sont soudées l'une à l'autre, sont disposées au moins des deux côtés des éléments élastiques (15, 17) dans la direction avant-arrière (LD) de manière à adhérer étroitement à ceux-ci, tandis que les parties au niveau desquelles les éléments élastiques (15, 17) croisent le site lié (80) constituent des parties fixées (84) au niveau desquelles les éléments élastiques (15, 17) sont fixés à la première feuille (12S) et à la deuxième feuille (12H) ;
l'une et/ou l'autre des régions de scellement latéral (12A) comporte la partie fixée (84) séparée latéralement d'un bord intérieur de la région de scellement latéral (12A) dans la direction de la largeur (WD), ainsi qu'un groupe de parties soudées (70) qui est au moins partiellement compris dans une zone de forme rectangulaire (RA) présentant, dans la direction avant-arrière (LD), une longueur de 1,5 mm vers le haut et de 1,5 mm vers le bas depuis la partie fixée (84) ;
les multiples parties soudées (70) sont disposées dans la direction de la largeur (WD) de telle sorte que, lorsque le groupe de parties soudées (70), qui est au moins partiellement compris dans la zone de forme rectangulaire (RA), est observé selon la direction avant-arrière (LD), une partie non soudée (72) présente, dans la direction de la largeur (WD), une dimension inférieure à 1,5 mm ; et
un diamètre maximal de chaque partie soudée est de 0,5 à 2,0 mm et un taux de surface des parties soudées (70) par rapport à la région de scellement latéral (12A) est de 5 à 30 %.

2. Article jetable de type culotte destiné à être porté selon la revendication 1,
dans lequel les multiples parties soudées (70) sont disposées dans la direction avant-arrière (LD) de telle sorte que, lorsque la totalité de la région de scellement latéral (12A) est observée selon la direction de la largeur (WD), une dimension de la partie non soudée (72) dans la direction avant-arrière (LD) est inférieure à une dimension de l'élément élastique (15, 17) dans la direction avant-arrière (LD) à l'état déployé.

3. Article jetable de type culotte destiné à être porté selon la revendication 2,
dans lequel des unités d'agencement des parties soudées (70) sont prévues de façon répétée à intervalles dans la direction avant-arrière (LD), et dans chacune des unités d'agencement, les parties soudées (70) sont disposées à intervalles le long d'une courbe en forme d'onde présentant une ligne centrale d'oscillation orientée selon la direction de la largeur (WD) et une longueur d'onde inférieure à une dimension de la région de scellement latéral (12A) dans la direction de la largeur (WD),
une partie soudée est prévue en chaque point auquel la courbe en forme d'onde croise la ligne centrale d'oscillation, et au moins une partie soudée est prévue de chaque côté de la ligne centrale d'oscillation dans la direction avant-arrière (LD).

4. Article jetable de type culotte destiné à être porté selon la revendication 3,
dans lequel les parties soudées (70), qui viennent en contact avec un bord latéral de la région de scellement latéral (12A), sont prévues de façon répétée à intervalles dans la direction avant-arrière (LD).

5. Article jetable de type culotte destiné à être porté selon l'une quelconque des revendications 1 à 4,
dans lequel un intervalle entre deux sites liés (80) adjacents dans la direction de la largeur (WD) est inférieur à une dimension de la région de scellement latéral (12A) dans la direction de la largeur (WD), et
le site lié (80) est formé selon une courbe en forme d'onde présentant une ligne centrale d'oscillation orientée selon la direction avant-arrière (LD) et une amplitude totale inférieure à la dimension de la région de scellement latéral (12A) dans la direction de la largeur (WD).
